(19) Europäisches Patentamt
European Patent Office
Office européen des brevets

(11) Veröffentlichungsnummer: **0 046 590**
**B1**

(12) # EUROPÄISCHE PATENTSCHRIFT

(45) Veröffentlichungstag der Patentschrift: **22.05.85**

(21) Anmeldenummer: **81106556.4**

(22) Anmeldetag: **24.08.81**

(51) Int. Cl.⁴: **C 07 D 303/48,** A 61 K 31/335, C 07 C 69/734 // C07C69/708, C07C43/225, C07C79/35, C07C43/23, C07C43/174, C07C43/178, C07C57/58, C07C21/24, C07C33/46

(54) **Phen(alk)oxy-substituierte Oxirancarbonsäuren, Verfahren zu ihrer Herstellung, ihre Anwendung und sie enthaltende Arzneimittel.**

(30) Priorität: **25.08.80 CH 6397/80**
**06.03.81 CH 1526/81**

(43) Veröffentlichungstag der Anmeldung: **03.03.82 Patentblatt 82/9**

(45) Bekanntmachung des Hinweises auf die Patenterteilung: **22.05.85 Patentblatt 85/21**

(84) Benannte Vertragsstaaten: **AT BE CH DE FR GB IT LI LU NL SE**

(56) Entgegenhaltungen:
**EP - A - 0 025 192**
**GB - A - 1 551 078**

**E. SCHRÖDER et al., *Arzneimittelchemie II*, Stuttgart, 1976, S. 187-191**

(73) Patentinhaber: **Byk Gulden Lomberg Chemische Fabrik GmbH, Byk-Gulden-Strasse 2, D-7750 Konstanz (DE)**

(72) Erfinder: **Eistetter, Klaus, Dr., Säntisblick 7, D-7750 Konstanz 19 (DE)**
Erfinder: **Ludwig, Gerhard, Dr., Meisenweg 6, D-7750 Konstanz (DE)**
Erfinder: **Rapp, Erich, Dr., Pfarrer-Braun-Strasse 6, D-7760 Radolfzell 17 (DE)**
Erfinder: **Wolf, Horst, Dr., Brandesstrasse 29, D-7750 Konstanz (DE)**

**EP 0 046 590 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft Phen(alk)oxy-substituierte Oxirancarbonsäuren, Verfahren zu ihrer Herstellung, ihre Anwendung und sie enthaltende Arzneimittel.

Phenoxymethyloxirane, z.B. 2-(4-Nitrophenoxymethyl)-oxiran, und Phenyloxirancarbonsäureester, z.B. 2-Phenyloxiran-2-carbonsäureethylester, wurden u.a. im Rahmen einer Untersuchung über die Fähigkeit von substituierten Dreiringverbindungen eingesetzt, als Substrat oder Inhibitor für Meerschweinchen-Lebermicrosomen-Epoxidhydrase zu dienen [F. Oesch et al., Biochem. 10(1971) No. 26, 4858-66]. Substituierte Glycidsäurederivate werden beschrieben in der US-PS 4 132 719, wobei der 2-ständige Substituent ein langkettiger Dibromalkylrest mit 11 bis 16 Kohlenstoffatomen sein soll; in der US-PS 4 132 720, wobei der 2-ständige Substituent ein langkettiger Alkenylrest mit 11 bis 16 Kohlenstoffatomen sein soll; in der US-PS 4 196 300 bzw. der GB-PS 1 551 078, wobei der 2-ständige Substituent ein langkettiger Alkylrest mit 11 bis 16 Kohlenstoffatomen sein soll.

Gegenstand der Erfindung sind Phen(alk)oxy-substituierte Oxirancarbonsäuren der allgemeinen Formel I

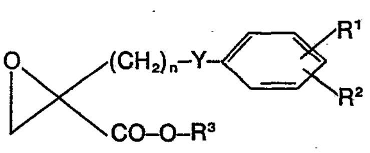

(I),

worin

$R^1$ ein Wasserstoffatom, ein Halogenatom, eine C1–C4-Niederalkylgruppe, eine C1–C4-Niederalkoxygruppe, eine Nitrogruppe oder eine Trifluormethylgruppe bedeutet,

$R^2$ eine der Bedeutungen von $R^1$ hat,

$R^3$ ein Wasserstoffatom oder eine C1–C4-Niederalkylgruppe,

Y die Gruppierung $-O-(CH_2)_m-$,

m 0 oder eine ganze Zahl von 1 bis 4 und

n eine ganze Zahl von 2 bis 8 bedeuten,

wobei die Summe von m und n eine ganze Zahl von 2 bis 8 ist, sowie die Salze der Carbonsäuren.

Die C1–C4-Niederalkylreste können geradkettig oder verzweigt sein. Geradkettige Alkylreste sind beispielsweise der Methyl-, Ethyl-, n-Propyl- und n-Butylrest, von denen die mit 1 und 2 Kohlenstoffatomen bevorzugt sind. Verzweigte Alkylreste sind beispielsweise der Isopropyl-, Isobutyl- und sek.-Butylrest, von denen der mit 3 Kohlenstoffatomen bevorzugt ist. Als Alkylreste von Niederalkoxygruppen kommen sowohl geradkettige als auch verzweigte Niederalkylgruppen in Frage. Die Methoxygruppe ist als Niederalkoxygruppe bevorzugt.

Halogenatome sind Fluor-, Chlor- und Bromatome, von denen Fluor, insbesondere Chlor bevorzugt sind.

Die Substituenten $R^1$ und $R^2$ des Phenylrings befinden sich bevorzugt in meta- oder para-Stellung zum (Alk)oxyalkylenoxirancarbonsäurerest.

Als Salze kommen Salze mit anorganischen und organischen Basen in Betracht. Pharmakologisch nicht verträgliche Salze werden nach an sich bekannten Methoden in pharmakologisch, d.h. biologisch, verträgliche Salze übergeführt, die unter den erfindungsgemässen Salzen bevorzugt sind. Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker, basische Aminosäuren, etc. zur Anwendung.

Beispielsweise seien die Salze von Lithium, Natrium, Kalium, Magnesium, Calcium, Aluminium, Ethylendiamin, Dimethylamin, Diethylamin, Morpholin, Piperidin, Piperazin, N-Niedrigalkylpiperazin (z.B. N-Methylpiperazin), Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methylglucamin, Glucosamin, N-Methylglucosamin, Lysin, Ornithin, Arginin, Chinolin genannt.

Eine Ausgestaltung der Erfindung sind Phen(alk)oxy-substituierte Oxirancarbonsäuren der allgemeinen Formel I*

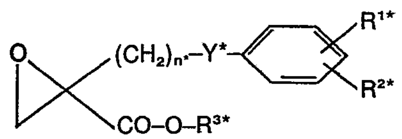

(I*),

worin

$R^{1*}$ und $R^{2*}$ meta- oder para-ständig sind und

$R^{1*}$ ein Wasserstoffatom, ein Chloratom, eine Methylgruppe, eine Methoxygruppe, eine Nitrogruppe oder eine Trifluormethylgruppe,

$R^{2*}$ ein Wasserstoffatom oder ein Chloratom,

$R^{3*}$ ein Wasserstoffatom oder eine C1–C4-Niedrigalkylgruppe,

Y* die Gruppierung $-O-(CH_2)_{m^*}-$,

m* 0 oder 1 und

n* eine ganze Zahl von 3 bis 7 bedeuten,

wobei die Summe von m* und n* eine ganze Zahl von 3 bis 7 ist, sowie die Salze der Carbonsäuren.

Eine bevorzugte Ausgestaltung der Erfindung sind Phenoxy-substituierte Oxirancarbonsäuren der allgemeinen Formel I**

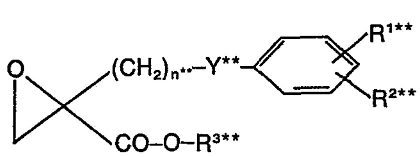

(I**),

worin

$R^{1**}$ meta- oder para-ständig ist und

$R^{1**}$ ein Wasserstoffatom, ein Chloratom oder eine Trifluormethylgruppe,

$R^{2**}$ ein Wasserstoffatom,

$R^{3**}$ ein Wasserstoffatom, eine Methylgruppe oder eine Ethylgruppe,

Y** die Gruppierung –O–,

n** eine ganze Zahl von 4 bis 6 bedeuten,

sowie die pharmakologisch verträglichen Salze

der Carbonsäuren mit anorganischen oder organischen Basen.

Als Vertreter der erfindungsgemässen Verbindungen seien beispielsweise

2-[2-(2-Phenylethoxy)-ethyl]-oxiran-2-carbonsäureethylester,
2-{2-[2-(4-Chlorphenyl)-ethoxy]-ethyl}-oxiran-2-carbonsäureisopropylester,
2-{2-[2-(4-Fluorphenyl)-ethoxy]-ethyl}-oxiran-2-carbonsäuremethylester,
2-{2-[2-(3-Trifluormethylphenyl)-ethoxy]-ethyl}-oxiran-2-carbonsäure-sek.-butylester,
2-{2-[3-(3-Chlorphenyl)-propoxy]-ethyl}-oxiran-2-carbonsäureethylester,
2-{2-[3-(4-Nitrophenyl)-propoxy]-ethyl}-oxiran-2-carbonsäuremethylester,
2-{2-[4-(3-Trifluormethylphenyl)-butoxy]-ethyl}-oxiran-2-carbonsäure-n-butylester,
2-{2-[4-(4-Chlorphenyl)-butoxy]-ethyl}-oxiran-2-carbonsäure-n-propylester,
2-{2-[4-(4-Bromphenyl)-butoxy]-ethyl}-oxiran-2-carbonsäureethylester,
2-[2-(3-Phenylpropoxy)-ethyl]-oxiran-2-carbonsäureethylester,
2-[2-(4-Phenylbutoxy)-ethyl]-oxiran-2-carbonsäureethylester,
2-[3-(2-Phenylethoxy)-propyl]-oxiran-2-carbonsäuremethylester,
2-[3-(3-Phenylpropoxy)-propyl]-oxiran-2-carbonsäureethylester,
2-[3-(4-Phenylbutoxy)-propyl]-oxiran-2-carbonsäure-n-propylester,
2-[4-(4-Phenylbutoxy)-butyl]-oxiran-2-carbonsäure-ethylester,
2-{3-[2-(4-Chlorphenyl)-ethoxy]-propyl}-oxiran-2-carbonsäureethylester,
2-{3-[2-(3-Trifluormethylphenyl)-ethoxy]-propyl}-oxiran-2-carbonsäuremethylester,
2-{3-[3-(4-Nitrophenyl)-propoxy]-propyl}-oxiran-2-carbonsäure-propylester,
2-{3-[4-(3-Trifluormethylphenyl)-butoxy]-propyl}-oxiran-2-carbonsäure-isopropylester,
2-{3-[4-(4-Chlorphenyl)-butoxy]-propyl}-oxiran-2-carbonsäureethylester,
2-{4-[2-(4-Chlorphenyl)-ethoxy]-butyl}-oxiran-2-carbonsäure-n-butylester,
2-{4-[2-(4-Fluorphenyl)-ethoxy]-butyl}-oxiran-2-carbonsäuremethylester,
2-{4-[2-(3-Trifluormethylphenyl)-ethoxy]-butyl}-oxiran-2-carbonsäureethylester,
2-{4-[3-(4-Nitrophenyl)-propoxy]-butyl}-oxiran-2-carbonsäureethylester,
2-{4-[4-(3-Trifluormethylphenyl)-butoxy]-butyl}-oxiran-2-carbonsäureethylester,
2-{4-[4-(4-Chlorphenyl)-butoxy]-butyl}-oxiran-2-carbonsäureethylester,
2-{5-[2-(4-Chlorphenyl)-ethoxy]-pentyl}-oxiran-2-carbonsäuremethylester,
2-{5-[2-(3-Trifluormethylphenyl)-ethoxy]-pentyl}-oxiran-2-carbonsäureethylester,
2-{5-[3-(4-Nitrophenyl)-propoxy]-pentyl}-oxiran-2-carbonsäureethylester,
2-{5-[3-(3-Chlorphenyl)-propoxy]-pentyl}-oxiran-2-carbonsäureethylester,
2-[5-(2-Phenylethoxy)-pentyl]-oxiran-2-carbonsäure-n-butylester,
2-[6-(2-Phenylethoxy)-hexyl]-oxiran-2-carbonsäureethylester,
2-{6-[2-(4-Chlorphenyl)-ethoxy]-hexyl}-oxiran-2-carbonsäure-n-propylester,
2-{6-[2-(4-Fluorphenyl)-ethoxy]-hexyl}-oxiran-2-carbonsäuremethylester,
2-{6-[2-(3-Trifluormethylphenyl)-ethoxy]-hexyl}-oxiran-2-carbonsäureethylester,
2-[2-(4-Chlorbenzyloxy)-ethyl]-oxiran-2-carbonsäureethylester,
2-[3-(3-Chlorbenzyloxy)-propyl]-oxiran-2-carbonsäuremethylester,
2-[4-(4-Fluorbenzyloxy)-butyl]-oxiran-2-carbonsäureisopropylester,
2-[2-(4-Bromphenoxy)-ethyl]-oxiran-2-carbonsäure-n-propylester,
2-[2-(4-Methoxyphenoxy)-ethyl]-oxiran-2-carbonsäureethylester,
2-[2-(4-Nitrophenoxy)-ethyl]-oxiran-2-carbonsäuremethylester,
2-[2-(4-Chlorphenoxy)-ethyl]-oxiran-2-carbonsäure-n-butylester,
2-[2-(4-Methoxyphenoxy)-ethyl]-oxiran-2-carbonsäuremethylester,
2-[2-(3-Trifluormethylphenoxy)-ethyl]-oxiran-2-carbonsäureethylester,
2-[3-(3-Fluorphenoxy)-propyl]-oxiran-2-carbonsäureethylester,
2-[3-(4-Bromphenoxy)-propyl]-oxiran-2-carbonsäuremethylester,
2-[3-(3-Methylphenoxy)-propyl]-oxiran-2-carbonsäure-sek.-butylester,
2-[3-(4-n-Butoxyphenoxy)-propyl]-oxiran-2-carbonsäureethylester,
2-[3-(2-Isopropylphenoxy)-propyl]-oxiran-2-carbonsäureisopropylester,
2-[3-(4-Chlorphenoxy)-propyl]-oxiran-2-carbonsäuremethylester,
2-[3-(3-Trifluormethylphenoxy)-propyl]-oxiran-2-carbonsäure-n-butylester,
2-[4-(4-Bromphenoxy)-butyl]-oxiran-2-carbonsäureethylester,
2-[4-(4-Chlorphenoxy)-butyl]-oxiran-2-carbonsäure-n-propylester,
2-[4-(3,4-Dichlorphenoxy)-butyl]-oxiran-2-carbonsäureethylester,
2-[4-(3-Chlor-4-methylphenoxy)-butyl]-oxiran-2-carbonsäuremethylester,
2-[4-(4-Methylphenoxy)-butyl]-oxiran-2-carbonsäureethylester,
2-[5-(2,5-Dimethoxyphenoxy)-pentyl]-oxiran-2-carbonsäureethylester,
2-[5-(3-Trifluormethylphenoxy)-pentyl]-oxiran-2-carbonsäure-n-butylester,
2-[5-(4-Methylphenoxy)-pentyl]-oxiran-2-carbonsäuremethylester,
2-[5-(3-Chlorphenoxy)-pentyl]-oxiran-2-carbonsäureisobutylester,
2-[5-(4-Nitrophenoxy)-pentyl]-oxiran-2-carbonsäureethylester,
2-[6-(4-Fluorphenoxy)-hexyl]-oxiran-2-carbonsäureethylester,

2-[6-(3-Trifluormethylphenoxy)-hexyl]-oxiran-2-carbonsäuremethylester,
2-[6-(3,4-Dichlorphenoxy)-hexyl]-oxiran-2-carbonsäure-n-butylester,
2-[6-(4-Chlorphenoxy)-hexyl]-oxiran-2-carbonsäureisopropylester,
2-[7-(3-Fluorphenoxy)-heptyl]-oxiran-2-carbonsäureethylester,
2-[7-(4-Trifluormethylphenoxy)-heptyl]-oxiran-2-carbonsäureethylester,
2-[7-(3-Chlor-4-methylphenoxy)-heptyl]-oxiran-2-carbonsäuremethylester,
2-[7-(3-Chlorphenoxy)-heptyl]-oxiran-2-carbonsäure-n-propylester,
2-[8-(4-Fluorphenoxy)-octyl]-oxiran-2-carbonsäureethylester,
2-[8-(3-Trifluormethylphenoxy)-octyl]-oxiran-2-carbonsäuremethylester,
2-[8-(3,4-Dichlorphenoxy)-octyl]-oxiran-2-carbonsäureethylester,
2-[8-(4-Chlorphenoxy)-octyl]-oxiran-2-carbonsäureisobutylester,
die entsprechenden Oxiran-2-carbonsäuren sowie deren Salze mit anorganischen und organischen Basen genannt.

Bevorzugte Vertreter sind
2-[4-(3-Chlorphenoxy)-butyl]-oxiran-2-carbonsäureethylester,
2-[4-(3-Trifluormethylphenoxy)-butyl]-oxiran-2-carbonsäureethylester,
2-[6-(4-Chlorphenoxy)-hexyl]-oxiran-2-carbonsäureethylester,
2-[5-(4-Chlorphenoxy)-pentyl]-oxiran-2-carbonsäureethylester,
die entsprechenden Oxiran-2-carbonsäuren sowie deren pharmakologisch verträgliche Salze.

Die Phen(alk)oxy-substituierten Oxirancarbonsäuren der allgemeinen Formel I bzw. der Ausgestaltungen I* und I** besitzen ein Chiralitätszentrum. Die Erfindung schliesst daher sowohl die Racemate und die Enantiomeren als auch deren Gemische ein.

Die erfindungsgemässen Verbindungen weisen wertvolle pharmakologische Eigenschaften auf, die sie gewerblich verwertbar machen. Sie wirken hypoglycämisch und hypoketonämisch.

Aufgrund ihrer vorteilhaften Wirksamkeit sind die erfindungsgemässen Phen(alk)oxy-substituierten Oxirancarbonsäuren der allgemeinen Formel I bzw. der Ausgestaltungen I* und I** sowie die pharmakologisch verträglichen Salze zur human- und veterinärmedizinischen Behandlung und Prophylaxe von Krankheiten, die auf Störungen des Glucose- und Fettstoffwechsels beruhen, geeignet. Beispielsweise werden behandelt prädiabetische Zustände zur Verhinderung der Manifestation des Diabetes, manifester Diabetes, z.B. Erwachsenendiabetes, labiler Diabetes von Jugendlichen sowie alle krankhaften Zustände, die mit einer pathologisch erhöhten Ketonkörperproduktion einhergehen.

Gegenstand der Erfindung sind daher auch die erfindungsgemässen Verbindungen zur Anwendung bei der Behandlung und Prophylaxe der angegebenen Krankheiten.

Ein weiterer Gegenstand der Erfindung sind Arzneimittel, die eine oder mehrere der Phen(alk)oxy-substituierten Oxirancarbonsäuren der allgemeinen Formel I

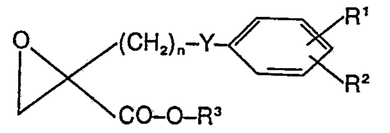
(I),

worin

$R^1$ ein Wasserstoffatom, ein Halogenatom, eine C1–C4-Niederalkylgruppe, eine C1–C4-Niederalkoxygruppe, eine Nitrogruppe oder eine Trifluormethylgruppe bedeutet,

$R^2$ eine der Bedeutungen von $R^1$ hat,

$R^3$ ein Wasserstoffatom oder eine C1–C4-Niederalkylgruppe,

Y die Gruppierung $-O-(CH_2)_m-$,

m 0 oder eine ganze Zahl von 1 bis 4 und

n eine ganze Zahl von 2 bis 8 bedeuten,

wobei die Summe von m und n eine ganze Zahl von 2 bis 8 ist, und/oder die pharmakologisch verträglichen Salze der Säuren mit anorganischen oder organischen Basen enthalten.

Ausgestaltungen der Arzneimittel sind solche, die Phen(alk)oxy-substituierte Oxirancarbonsäuren der Ausgestaltungen I* oder I** und/oder die pharmakologisch verträglichen Salze der Säuren mit anorganischen oder organischen Basen, enthalten.

Gegenstand der Erfindung ist ausserdem die Verwendung der erfindungsgemässen Verbindungen zur Herstellung von Arzneimitteln zur Bekämpfung der angegebenen Krankheiten.

Die Arzneimittel werden nach an sich bekannten Verfahren hergestellt. Als Arzneimittel werden die erfindungsgemässen Verbindungen entweder als solche oder gegebenenfalls in Kombination mit geeigneten pharmazeutischen Trägerstoffen eingesetzt. Enthalten die pharmazeutischen Zubereitungen neben den Wirkstoffen pharmazeutische Trägerstoffe, beträgt der Wirkstoffgehalt dieser Mischungen 1 bis 95, vorzugsweise 10 bis 85, Gewichtsprozent der Gesamtmischung. Die Arzneimittel werden beispielsweise für die orale oder parenterale (intravenöse, intramusculäre) Gabe in geeigneten Dosen formuliert. Die Tagesdosis für die orale Applikation an Menschen liegt im allgemeinen zwischen 0,1 und 30, vorzugsweise 0,3 bis 15, insbesondere 0,6 bis 3 mg/kg Körpergewicht. Die Dosierung für die parenterale Behandlung liegt zwischen 0,3 bis 1 mg Wirkstoff/kg Körpergewicht.

Die pharmazeutischen Zubereitungen bestehen bevorzugt aus den erfindungsgemässen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süssungsmittel, als Geschmackskorrigenz, als Farbstoff oder als Konservierungsmittel dienen.

Neben den erfindungsgemässen Phen(alk)oxy-substituierten Oxirancarbonsäuren I, in denen die Substituenten die oben angegebene Bedeutung haben, und/oder ihren Salzen können die pharmazeutischen Zubereitungen weiterhin einen oder mehrere pharmakologisch aktive Bestandteile anderer Arzneimittelgruppen, wie Antidiabetika (Sulfonamide, Sulfonylharnstoffe u.a.), z.B. Carbutamid, Tolbutamid, Chlorpropamid, Glibenclamid, Glibornurid, Glisoxepid, Gliquidon, Glymidin oder Hypolipidämika, wie Nikotinsäure sowie deren Derivate und Salze, enthalten.

Ein weiterer Gegenstand der Erfindung ist ein Verfahren zur Herstellung von Phen(alk)oxy-substituierten Oxirancarbonsäuren der allgemeinen Formel I

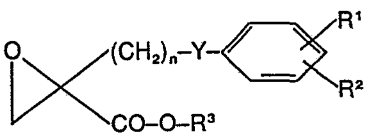

(I),

worin

$R^1$ ein Wasserstoffatom, ein Halogenatom, eine C1–C4-Niederalkylgruppe, eine C1–C4-Niederalkoxygruppe, eine Nitrogruppe oder eine Trifluormethylgruppe bedeutet,

$R^2$ eine der Bedeutungen von $R^1$ hat,

$R^3$ ein Wasserstoffatom oder eine C1–C4-Niederalkylgruppe,

Y die Gruppierung $-O-(CH_2)_m-$,

m 0 oder eine ganze Zahl von 1 bis 4 und

n eine ganze Zahl von 2 bis 8 bedeuten,

wobei die Summe von m und n eine ganze Zahl von 2 bis 8 ist, und den Salzen der Säuren, dadurch gekennzeichnet, dass man eine substituierte α-Methylencarbonsäure der allgemeinen Formel II

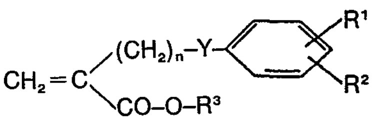

(II),

worin $R^1$, $R^2$, $R^3$, Y und n die vorstehend angegebene Bedeutung haben, oxidiert und gegebenenfalls anschliessend die erhaltenen Niederalkylester verseift oder die erhaltenen Säuren in die Salze oder Niederalkylester überführt.

Die Oxidation (Epoxidation) der α-Methylencarbonsäuren II erfolgt unter den Bedingungen, wie sie dem Fachmann für die Oxidation von Kohlenstoff-Kohlenstoff-Doppelbindungen zu Epoxiden bekannt sind. Als Oxidationsmittel kommen beispielsweise Peroxoverbindungen, wie Wasserstoffperoxid, Peressigsäure, Trifluorperessigsäure, 3,5-Dinitroperbenzoesäure, bevorzugt m-Chlorperbenzoesäure, Permaleinsäure in Betracht. Die Reaktion wird zweckmässigerweise in inerten Lösungsmitteln, z.B. aromatischen oder chlorierten Kohlenwasserstoffen, wie Benzol, Toluol, Dichlormethan, Chloroform durchgeführt. Die Reaktionstemperaturen liegen zwischen 0° und der Siedetemperatur des Lösungsmittels, bevorzugt zwischen 20° und 70 °C.

Die Verseifung der Niederalkylester erfolgt in an sich bekannter Weise. Sie wird beispielsweise mit einer wässerigen oder alkoholischen (z.B. ethanolischen) Alkalimetallhydroxid-(z.B. Kaliumhydroxid-)Lösung bei Raumtemperatur vorgenommen, gegebenenfalls unter Zusatz eines inerten Verdünnungsmittels, wie Dioxan, Tetrahydrofuran oder Toluol.

Die Überführung der Säuren der allgemeinen Formel I ($R^3$ = –H) bzw. der Ausgestaltungen I* und I** in die Salze kann durch direkte alkalische Hydrolyse der Säurederivate I ($R^3$ = Niederalkyl) erfolgen. Als alkalischer Reaktionspartner wird diejenige anorganische oder organische Base verwendet, deren Salz gewünscht wird. Man erhält die Salze aber auch, indem man die Säuren I ($R^3$ = –H) mit dem stöchiometrischen Äquivalent an entsprechender Base, z.B. Natriumhydroxid oder Natriumethanolat umsetzt, oder leicht lösliche Salze durch doppelte Umsetzung in schwer lösliche Salze überführt, oder beliebige Salze in pharmakologisch verträgliche Salze überführt.

Die Überführung der Oxirancarbonsäuren der allgemeinen Formel I ($R^3$ = –H) bzw. der Ausgestaltungen I* und I** in die Niederalkylester ($R^3$ = Niederalkyl) erfolgt in üblicher Weise. Beispielsweise werden sie mit Niederalkanolen in Gegenwart von starken Säuren, wie Schwefelsäure, p-Toluolsulfonsäure, oder von sauren Ionenaustauschern unter Bedingungen, bei denen keine Decarboxylierung stattfindet, oder mit Dialkylsulfaten oder Alkylhalogeniden in Gegenwart von Diazabicycloundecen oder Diazabicyclononen in inerten Lösungsmitteln, wie Benzol, Toluol, Aceton, verestert.

Die Verbindungen der allgemeinen Formel I fallen normalerweise in Form von racemischen Gemischen an, die mittels bekannter Verfahren in die Enantiomeren getrennt werden. Beispielsweise wandelt man mit einem optisch aktiven Spaltungsmittel das Racemat in Diastereomere um, die anschliessend durch selektive Kristallisation getrennt und in die entsprechenden optischen Isomeren übergeführt werden. Als optisch aktive Spaltungsmittel dienen z.B. optisch aktive Basen, wie 1- und d-1-Phenylethylamin, Cinchonidin oder d-Ephedrin, aus denen Salze der Säuren der allgemeinen Formel I, oder optisch aktive Alkohole, wie Borneol oder Menthol, aus denen Ester der Säuren der allgemeinen Formel I hergestellt werden. Man kann auch racemische Gemische durch Chromatographie über optisch aktive Sorptionsmittel in die optischen Isomeren zerlegen. Alternativ werden zunächst die α-Methylencarbonsäuren II mit einem optisch aktiven Spaltungsmittel umgesetzt, z.B. Borneol oder Menthol, die erhaltenen Produkte werden zu den entsprechenden Diastereomerengemischen der Oxirancarbonsäureester oxydiert, aus denen dann in üblicher Weise die optischen Isomeren der Säuren I gewonnen werden.

Zur Herstellung der Phen(alk)oxy-substituierten Oxirancarbonsäuren der Ausgestaltungen I* und I** werden α-Methylencarbonsäuren der allgemeinen Formeln II* und II**

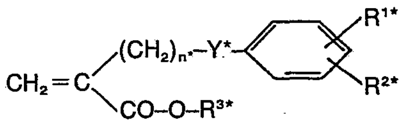
(II*)

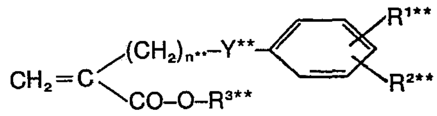
(II**)

worin $R^{1*}$, $R^{2*}$, $R^{3*}$, Y* und n* bzw. $R^{1**}$, $R^{2**}$, $R^{3**}$, Y** und n** die oben angegebene Bedeutung haben, eingesetzt.

Die α-Methylencarbonsäuren der allgemeinen Formeln II, II* und II** können nach an sich bekannten Methoden hergestellt werden. Sie sind wertvolle Zwischenprodukte für die Synthese der Oxirancarbonsäuren I, I* und I**.

Die Herstellung der α-Methylencarbonsäuren II und ihrer Salze erfolgt beispielsweise in Analogie zu H. Stetter und H. Kuhlmann [Synthesis 1979, 29] durch Umsetzung von Malonsäurehalbestern der allgemeinen Formel III

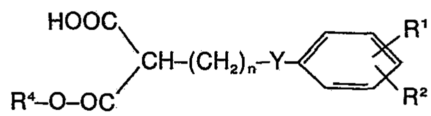
(III),

worin $R^1$, $R^2$, Y und n die oben angegebene Bedeutung haben und $R^4$ eine Niederalkylgruppe bedeutet, mit Formaldehyd in Pyridin in Gegenwart von sekundären Aminen, vorzugsweise Piperidin, und gegebenenfalls anschliessende Verseifung der erhaltenen Niederalkylester oder Umwandlung in die Salze.

Die Herstellung der Malonsäurehalbester III erfolgt nach Methoden wie sie dem Fachmann geläufig sind, beispielsweise durch Umsetzung von Dialkylmalonaten IV mit Phen(alk)oxyalkylverbindungen V und partielle Hydrolyse der erhaltenen Malonsäurediester VI nach folgendem Schema

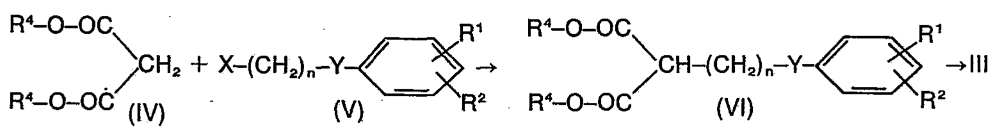
(IV) (V) (VI)

worin $R^1$, $R^2$, $R^4$, Y und n die oben angegebene Bedeutung haben und X eine Fluchtgruppe (Austrittsgruppe), z. B. ein Chlor- oder Bromatom, eine Mesyloxy- oder eine p-Toluolsulfonyloxygruppe, bedeutet.

Für die Herstellung der α-Methylencarbonsäuren II* bzw. II** werden entsprechende Ausgangsverbindungen III* bzw. III**

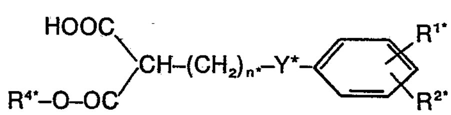
(III*),

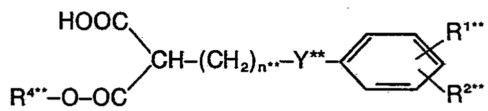
(III**),

IV* bzw. IV**

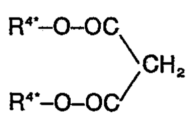
(IV*),

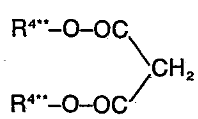
(IV**),

V* bzw. V**

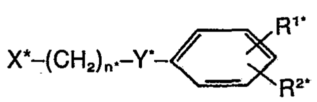
(V*),

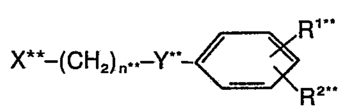
(V**),

VI* bzw. VI**

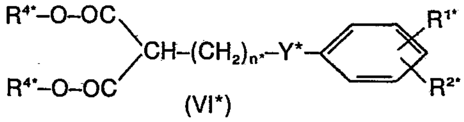
(VI*)

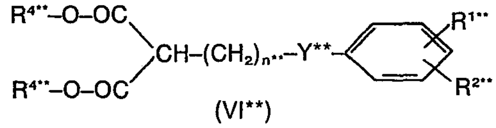
(VI**)

eingesetzt, in denen $R^{1*}$, $R^{2*}$, $Y^*$ und $n^*$ bzw. $R^{1**}$, $R^{2**}$, $Y^{**}$ und $n^{**}$ die oben angegebenen Bedeutungen haben; $R^{4*}$ eine Niedrigalkylgruppe, $R^{4**}$ eine Methyl- oder Ethylgruppe und $X^*$ bzw. $X^{**}$ ein Chlor- oder Bromatom, eine Mesyloxy- oder eine p-Toluolsulfonyloxygruppe bedeuten.

Die Phen(alk)oxyalkylverbindungen V bzw. deren Ausgestaltungen $V^*$ und $V^{**}$ werden nach bekannten Verfahren hergestellt, z.B. analog J. Augstein et al. [J. Med. Chem. 8 (1965) 356–367] oder J.D. Genzer et al. [J. Amer. Chem. Soc. 73 (1951) 3159–3162] oder Sh. Mamedov et al. [Chem. Abstr. 59 (1963) 4410e und Chem. Abstr. 60 (1964) 5321c]. Beispielsweise werden die Phen(alk)oxyalkylverbindungen V analog Genzer et al. durch Reaktion der Diole VII mit den Phenalkylchloriden VIII und Umsetzung der erhaltenen Phenalkoxyalkanole IX mit Thionylhalogeniden oder Sulfonsäurehalogeniden nach folgendem Schema hergestellt:

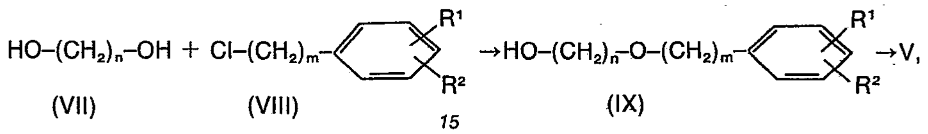

(VII) (VIII) (IX)

worin $R^1$, $R^2$ und n die oben angegebene Bedeutung haben und m eine ganze Zahl von 1 bis 4 bedeutet.

Die Phenalkylchloride VIII werden nach bekannten Verfahren durch Reduktion der Carbonsäuren X und anschliessende Umsetzung der erhaltenen Alkanole XI mit Thionylchlorid nach folgendem Schema hergestellt:

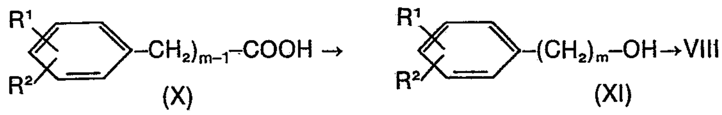

(X) (XI)

worin $R^1$ und $R^2$ die oben angegebene Bedeutung haben und m eine ganze Zahl von 1 bis 4 bedeutet.

Die folgenden Beispiele dienen zur Erläuterung der Erfindung. Kp. bedeutet Siedepunkt, F. bedeutet Schmelzpunkt. Temperaturangaben erfolgen in °C.

## Beispiele

### Beispiel 1

2-(4-Phenoxybutyl)-oxiran-2-carbonsäure-ethylester

a) 2-(4-Phenoxybutyl)-oxiran-2-carbonsäure-ethylester

32 g 2-Methylen-6-phenoxyhexansäureethylester und 78,5 g m-Chlorperbenzoesäure (85%ig) werden in 200 ml Methylenchlorid 24 Stunden unter Rückfluss gekocht. Man lässt abkühlen, filtriert von der abgeschiedenen m-Chlorbenzoesäure ab und wäscht gut mit Petrolether nach. Die vereinigten Filtrate werden eingeengt, der rotbraune ölige Rückstand in 150 ml Aceton gelöst und mit 100 ml gesättigter Natriumhydrogencarbonatlösung und 100 ml Wasser 30 Minuten durchgerührt. Man extrahiert anschliessend 3mal mit je 500 ml Petrolether und engt die vereinigten organischen Phasen ein. Der rotbraune ölige Rückstand wird im Vakuum destilliert, die bei 113–140 °C (0,008 Torr = 1,06 Pa) gesammelte Fraktion wird über eine Kieselgelsäule gereinigt (Laufmittel: Petrolether/Essigsäureethylester 9:1) und erneut destilliert. Man erhält 6,15 g der Titelverbindung vom Kp. 125° bei 0,07 Torr (9,3 Pa).

b) 2-Methylen-6-phenoxyhexansäureethylester

66,45 g 4-Phenoxybutylmalonsäureethylester, 45 ml Pyridin, 3 ml Piperidin und 9,9 g Paraformaldehyd werden 4 Stunden bei 50° gerührt. Die erkaltete Reaktionsmischung wird mit 400 ml Wasser versetzt, mit halbkonzentrierter Salzsäure unter Eiskühlung angesäuert, und 3mal mit je 200 ml Diethylether extrahiert. Die vereinigten organischen Phasen werden eingeengt und im Vakuum destilliert. Man erhält 43,6 g 2-Methylen-6-phenoxyhexansäureethylester als farblose Flüssigkeit vom Kp. 140° bei 0,06 Torr (8 Pa).

c) 4-Phenoxybutylmalonsäureethylester

Man tropft bei Raumtemperatur eine Lösung von 16,1 g Kaliumhydroxid in 250 ml Ethanol zu 87 g 4-Phenoxybutylmalonsäurediethylester in 250 ml Ethanol. Man rührt 24 Stunden, engt im Vakuum weitgehend ein, nimmt den Rückstand in 500 ml Wasser auf und extrahiert mit 200 ml Diethylether, säuert die wässerige Phase unter Eiskühlung mit konzentrierter Salzsäure an und extrahiert 3mal mit je 200 ml Diethylether. Die organische Phase wird nach dem Trocknen über Natriumsulfat eingeengt. Zurück bleiben 66,45 g 4-Phenoxybutylmalonsäureethylester vom F. 69–72°.

### Beispiel 2

2-[4-(4-Methylphenoxy)-butyl]-oxiran-2-carbonsäureethylester

a) 2-[4-(4-Methylphenoxy)-butyl]-oxiran-2-carbonsäureethylester

Nach der in Beispiel 1a) beschriebenen Arbeitsweise erhält man 23,3 g der Titelverbindung [als hellgelbes Öl, das durch Chromatographie an einer Kieselgelsäule (Laufmittel: Petrolether/Methylenchlorid 1:1) gereinigt wird; $R_f$ 0,4 bei Dünnschichtchromatographie an Kieselgel mit Chloroform] aus 116,6 g 6-(4-Methylphenoxy)-2-methylenhexansäureethylester und 152,2 g m-Chlorperbenzoesäure. 41,8 g der Ausgangsverbindung werden zurückgewonnen.

b) 6-(4-Methylphenoxy)-2-methylenhexansäure-ethylester

Nach der in Beispiel 1b) beschriebenen Arbeitsweise erhält man 166,6 g 6-(4-Methylphenoxy)-2-methylenhexansäureethylester [vom Kp.

132–134 °C bei 0,1 Torr (13,3 Pa)] aus 254 g 4-(4-Methylphenoxy)-butylmalonsäureethylester, 27,2 g Paraformaldehyd, 260 ml Pyridin und 9 ml Piperidin.

c) 4-(4-Methylphenoxy)-butylmalonsäureethylester

Nach der in Beispiel 1c) beschriebenen Arbeitsweise erhält man 254,2 g 4-(4-Methylphenoxy)-butylmalonsäureethylester als hellgelbes Öl aus 303,7 g 4-(4-Methylphenoxy)-butylmalonsäurediethylester und 60,8 g Kaliumhydroxid in 1,5 l Ethanol.

d) 4-(4-Methylphenoxy)-butylmalonsäurediethylester

Man tropft bei 50 °C 176,2 g Malonsäurediethylester zu einer aus 23 g Natrium und 500 ml Ethanol frisch hergestellten Natriumethylatlösung. Man hält noch 2 Stunden bei dieser Temperatur und tropft anschliessend 243,2 g 4-(4-Methylphenoxy)-butylbromid zu. Nach beendeter Zugabe rührt man 3 Stunden bei 60 °C, engt dann weitgehend ein, versetzt den Rückstand mit 1 l Eiswasser und extrahiert 3mal mit insgesamt 1 l Methylenchlorid. Die vereinigten organischen Phasen werden über Natriumsulfat getrocknet und das Lösungsmittel sowie überschüssiger Malonsäurediethylester im Vakuum abdestilliert. Man erhält 303,9 g 4-(4-Methylphenoxy)-butylmalonsäurediethylester als hellgelben, öligen Rückstand.

Beispiel 3

2-[4-(3-Trifluormethylphenoxy)-butyl]-oxiran-2-carbonsäureethylester

a) 2-[4-(3-Trifluormethylphenoxy)-butyl]-oxiran-2-carbonsäureethylester

Nach der in Beispiel 1a) beschriebenen Arbeitsweise erhält man 46,9 g der Titelverbindung [als farbloses Öl, gereinigt durch Chromatographie an Kieselgel (Laufmittel: Petrolether/Methylenchlorid 1:1); $R_f$ 0,6 bei Dünnschichtchromatographie an Kieselgel mit Petrolether/Essigsäureethylester/Essigsäure (80:20:3)] aus 48,3 g 2-Methylen-6-(3-trifluormethylphenoxy)-hexansäureethylester und 62 g m-Chlorperbenzoesäure in 950 ml Methylenchlorid.

b) 2-Methylen-6-(3-trifluormethylphenoxy)-hexansäureethylester

Nach der in Beispiel 1b) beschriebenen Arbeitsweise erhält man 58,7 g 2-Methylen-6-(3-trifluormethylphenoxy)-hexansäureethylester [als farbloses Öl, gereinigt durch Chromatographie an Kieselgel (Laufmittel: Petrolether/Methylenchlorid 1:1)] aus 90,7 g 4-(3-Trifluormethylphenoxy)-butylmalonsäureethylester, 8,3 g Paraformaldehyd, 95 ml Pyridin und 2,5 ml Piperidin.

c) 4-(3-Trifluormethylphenoxy)-butylmalonsäureethylester

Nach der in Beispiel 1c) beschriebenen Arbeitsweise erhält man 90,7 g 4-(3-Trifluormethylphenoxy)-butylmalonsäureethylester (als gelbliches, zähes Öl) aus 117 g 4-(3-Trifluormethylphenoxy)-butylmalonsäurediethylester und 20,5 g Kaliumhydroxid in 650 ml Ethanol.

d) 4-(3-Trifluormethylphenoxy)-butylmalonsäurediethylester

Nach der in Beispiel 2d) beschriebenen Arbeitsweise erhält man 117,4 g 4-(3-Trifluormethylphenoxy)-butylmalonsäurediethylester (als helles Öl) aus 102,5 g 4-(3-Trifluormethylphenoxy)-butylbromid, 58 g Malonsäurediethylester und einer Lösung von 8 g Natrium in 280 ml Ethanol.

e) 4-(3-Trifluormethylphenoxy)-butylbromid

267 ml 1,6 n Natronlauge werden bei 100° zu 69,3 g 3-Hydroxybenzotrifluorid und 119 g 1,4-Dibrombutan innerhalb 1,5 Stunden unter Rühren zugetropft. Man rührt noch 5 Stunden bei dieser Temperatur nach, lässt abkühlen und versetzt mit 200 ml Methylenchlorid. Die organische Phase wird abgetrennt, mit verdünnter Natronlauge gewaschen, über Natriumsulfat getrocknet und eingeengt. Der Rückstand wird im Vakuum destilliert. Man erhält 102,6 g 4-(3-Trifluormethylphenoxy)-butylbromid vom Kp. 143–144° bei 13 Torr (1730 Pa).

Beispiel 4

2-[4-(3-Chlorphenoxy)-butyl]-oxiran-2-carbonsäureethylester

a) 2-[4-(3-Chlorphenoxy)-butyl]-oxiran-2-carbonsäureethylester

Nach der in Beispiel 1a) beschriebenen Arbeitsweise erhält man 50,7 g der Titelverbindung [als farbloses Öl vom Kp. 135–136° bei 0,05 Torr (6,7 Pa)] aus 110,2 g 6-(3-Chlorphenoxy)-2-methylenhexansäureethylester und 135,5 g m-Chlorperbenzoesäure in 1,4 l Methylenchlorid.

b) 6-(3-Chlorphenoxy)-2-methylenhexansäureethylester

Nach der in Beispiel 1b) beschriebenen Arbeitsweise erhält man 110,6 g 6-(3-Chlorphenoxy)-2-methylenhexansäureethylester [vom Kp. 131–134° bei 0,1 Torr (13,3 Pa)] aus 178 g 4-(3-Chlorphenoxy)-butylmalonsäureethylester, 17,8 g Paraformaldehyd, 180 ml Pyridin und 5,6 ml Piperidin.

c) 4-(3-Chlorphenoxy)-butylmalonsäureethylester

Nach der in Beispiel 1c) beschriebenen Arbeitsweise erhält man 178,2 g 4-(3-Chlorphenoxy)-butylmalonsäureethylester (als hellgelbes Öl) aus 231,5 g 4-(3-Chlorphenoxy)-butylmalonsäurediethylester und 43,6 g Kaliumhydroxid in 1,05 l Ethanol.

d) 4-(3-Chlorphenoxy)-butylmalonsäurediethylester

Nach der in Beispiel 2d) beschriebenen Arbeitsweise erhält man 231,7 g 4-(3-Chlorphenoxy)-butylmalonsäurediethylester (als hellgelbes Öl) aus 186,9 g 4-(3-Chlorphenoxy)-butylbromid, 124,9 g Malonsäurediethylester und einer Lösung von 16,3 g Natrium in 650 ml Ethanol.

Beispiel 5
2-(2-Phenoxyethyl)-oxiran-2-carbonsäure-ethylester

a) 2-(2-Phenoxyethyl)-oxiran-2-carbonsäure-ethylester

Nach der in Beispiel 1a) beschriebenen Arbeitsweise erhält man 9,3 g der Titelverbindung [als fast farbloses Öl, gereinigt durch Chromatographie über eine Kieselgelsäule (Laufmittel: Methylenchlorid); $R_f$ 0,4 bei Dünnschichtchromatographie an Kieselgel mit Petrolether/Essigsäureethylester/Essigsäure (80:20:3)] aus 25,4 g 2-Methylen-4-phenoxybuttersäureethylester und 43,7 g m-Chlorperbenzoesäure in 350 ml Methylenchlorid.

b) 2-Methylen-4-phenoxybuttersäureethylester

Nach der in Beispiel 1b) beschriebenen Arbeitsweise erhält man 62,5 g 2-Methylen-4-phenoxybuttersäureethylester [als farbloses Öl vom Kp. 97–100° bei 0,01 Torr (1,3 Pa)] aus 94,5 g 2-Phenoxyethylmalonsäureethylester, 11,8 g Paraformaldehyd, 95 ml Pyridin und 3,8 ml Piperidin.

c) 2-Phenoxyethylmalonsäureethylester

Nach der in Beispiel 1c) beschriebenen Arbeitsweise erhält man 94,5 g 2-(Phenoxy)-ethylmalonsäureethylester aus 147 g 2-Phenoxyethylmalonsäurediethylester und 28,1 g Kaliumhydroxid in 800 ml Ethanol.

Beispiel 6
2-(3-Phenoxypropyl)-oxiran-2-carbonsäure-ethylester

a) 2-(3-Phenoxypropyl)-oxiran-2-carbonsäure-ethylester

Nach der in Beispiel 1a) beschriebenen Arbeitsweise erhält man 14,0 g der Titelverbindung [als fast farbloses Öl, gereinigt durch Chromatographie über eine Kieselgelsäule (Laufmittel: Methylenchlorid); $R_f$ 0,4 bei Dünnschichtchromatographie an einer Kieselgelplatte mit Petrolether/Essigsäureethylester/Essigsäure (80:20:3)] aus 70,3 g 2-Methylen-5-phenoxyvaleriansäureethylester und 124,3 g m-Chlorperbenzoesäure in 900 ml Methylenchlorid.

b) 2-Methylen-5-phenoxyvaleriansäureethylester

Nach der in Beispiel 1b) beschriebenen Arbeitsweise erhält man 142,4 g 2-Methylen-5-phenoxyvaleriansäureethylester [vom Kp. 108–111° bei 0,01 Torr (1,3 Pa)] aus 200,6 g 3-Phenoxypropylmalonsäureethylester, 23,8 g Paraformaldehyd, 200 ml Pyridin und 7,6 ml Piperidin.

c) 3-Phenoxypropylmalonsäureethylester

Nach der in Beispiel 1c) beschriebenen Arbeitsweise erhält man 200,6 g 3-Phenoxypropylmalonsäureethylester (als gelbes Öl) aus 302 g 3-Phenoxypropylmalonsäurediethylester und 52,2 g Kaliumhydroxid in 1,2 l Ethanol.

Beispiel 7
2-[6-(4-Chlorphenoxy)-hexyl]-oxiran-2-carbonsäureethylester

a) 2-[6-(4-Chlorphenoxy)-hexyl]-oxiran-2-carbonsäureethylester

Nach der in Beispiel 1a) beschriebenen Arbeitsweise erhält man 5,5 g der Titelverbindung [als farbloses Öl vom Kp. 164 °C bei 0,2 Torr (26,6 Pa)] aus 15,0 g 8-(4-Chlorphenoxy)-2-methylenoctansäureethylester und 14,2 g m-Chlorperbenzoesäure in 50 ml Methylenchlorid.

b) 8-(4-Chlorphenoxy)-2-methylenoctansäure-ethylester

Nach der in Beispiel 1b) beschriebenen Arbeitsweise erhält man 52,1 g 8-(4-Chlorphenoxy)-2-methylenoctansäureethylester [vom Kp. 171° bei 0,1 Torr (13,3 Pa)] aus 74 g 6-(4-Chlorphenoxy)-hexylmalonsäureethylester, 8,2 g Paraformaldehyd, 41 ml Pyridin und 2,8 ml Piperidin.

c) 6-(4-Chlorphenoxy)-hexylmalonsäureethylester

Nach der in Beispiel 1c) beschriebenen Arbeitsweise erhält man 74 g 6-(4-Chlorphenoxy)-hexylmalonsäureethylester (als gelbliches zähes Öl) aus 83,5 g 6-(4-Chlorphenoxy)-hexylmalonsäurediethylester und 14,4 g Kaliumhydroxid in 500 ml Ethanol.

d) 6-(4-Chlorphenoxy)-hexylmalonsäure-diethylester

Nach der in Beispiel 2d) beschriebenen Arbeitsweise erhält man 87 g 6-(4-Chlorphenoxy)-hexylmalonsäurediethylester [vom Kp. 173° bei 0,1 Torr (13,3 Pa)] aus 106,7 g 6-(4-Chlorphenoxy)-hexylbromid, 53,8 g Malonsäurediethylester und einer Lösung von 8,4 g Natrium in 350 ml Ethanol.

Beispiel 8
2-[3-(4-Nitrophenoxy)-propyl]-oxiran-2-carbonsäureethylester

a) 2-[3-(4-Nitrophenoxy)-propyl]-oxiran-2-carbonsäureethylester

Nach der in Beispiel 1a) beschriebenen Arbeitsweise erhält man 3,8 g 2-[3-(4-Nitrophenoxy)-propyl]-oxiran-2-carbonsäureethylester (als gelbes Öl, das nach längerem Stehen erstarrt, F. 60°) aus 10 g 2-Methylen-5-(4-nitrophenoxy)-valeriansäureethylester und 10,5 g m-Chlorperbenzoesäure in 50 ml Methylenchlorid.

b) 2-Methylen-5-(4-nitrophenoxy)-valeriansäureethylester

Nach der in Beispiel 1b) beschriebenen Arbeitsweise erhält man 61 g 2-Methylen-5-(4-nitrophenoxy)-valeriansäureethylester [als gelbes Öl vom Kp. 167–170° bei 0,005 Torr (0,7 Pa)] aus 89 g 3-(4-Nitrophenoxy)-propylmalonsäureethylester, 10,8 g Paraformaldehyd, 54 ml Pyridin und 4 ml Piperidin.

c) 3-(4-Nitrophenoxy)-propylmalonsäureethylester

Nach der in Beispiel 1c) beschriebenen Arbeitsweise erhält man 90 g 3-(4-Nitrophenoxy)-propylmalonsäureethylester (als gelbes Öl) aus 116,5 g 3-(4-Nitrophenoxy)-propylmalonsäurediethylester und 21,7 g Kaliumhydroxid in 500 ml Ethanol.

d) 3-(4-Nitrophenoxy)-propylmalonsäure-diethylester

Nach der in Beispiel 2d) beschriebenen Arbeitsweise erhält man 119,5 g 3-(4-Nitrophenoxy)-propylmalonsäurediethylester (als gelbliches Öl) aus 110,5 g 3-(4-Nitrophenoxy)-propylbromid, 101 g Malonsäurediethylester und einer Lösung von 9,7 g Natrium in 1,1 l Ethanol.

Beispiel 9

2-(5-Phenoxypentyl)-oxiran-2-carbonsäure-ethylester

a) 2-(5-Phenoxypentyl)-oxiran-2-carbonsäure-ethylester

Nach der in Beispiel 1a) beschriebenen Arbeitsweise erhält man 2,15 g der Titelverbindung [als farbloses Öl, gereinigt durch Chromatographie an Kieselgel (Laufmittel: Petrolether/Essigsäureethylester 9:1); Dünnschichtchromatographie an Silicagel: $R_f$ 0,4] aus 10 g 2-Methylen-7-phenoxyheptansäureethylester und 11,6 g m-Chlorperbenzoesäure in 50 ml Methylenchlorid. 4,7 g der Ausgangsverbindung werden zurückgewonnen.

b) 2-Methylen-7-phenoxyheptansäureethylester

Nach der in Beispiel 1b) beschriebenen Arbeitsweise erhält man 58 g 2-Methylen-7-phenoxyheptansäureethylester [vom Kp. 118° bei 0,001 Torr (0,13 Pa)] aus 143,7 g 5-Phenoxypentylmalonsäureethylester, 18,3 g Paraformaldehyd, 92 ml Pyridin und 6 ml Piperidin.

c) 5-Phenoxypentylmalonsäureethylester

Nach der in Beispiel 1c) beschriebenen Arbeitsweise erhält man 143,7 g 5-Phenoxypentylmalonsäureethylester (als gelbliches Öl) aus 174,4 g 5-Phenoxypentylmalonsäurediethylester und 34,4 g Kaliumhydroxid in 500 ml Ethanol.

Beispiel 10

2-[5-(4-Chlorphenoxy)-pentyl]-oxiran-2-carbonsäureethylester

a) 2-[5-(4-Chlorphenoxy)-pentyl]-oxiran-2-carbonsäureethylester

Nach der in Beispiel 1a) beschriebenen Arbeitsweise erhält man 6,6 g der Titelverbindung [vom Kp. 176–178° bei 0,005 Torr (0,7 Pa)] aus 10 g 7-(4-Chlorphenoxy)-2-methylenheptansäureethylester und 10,9 g m-Chlorperbenzoesäure in 50 ml Methylenchlorid.

b) 7-(4-Chlorphenoxy)-2-methylenheptansäure-ethylester

Nach der in Beispiel 1b) beschriebenen Arbeitsweise erhält man 88,6 g 7-(4-Chlorphenoxy)-2-methylenheptansäureethylester [vom Kp. 154° bei 0,01 Torr (1,3 Pa)] aus 202,5 g 5-(4-Chlorphenoxy)-pentylmalonsäureethylester, 23,4 g Paraformaldehyd, 116 ml Pyridin und 7,9 ml Piperidin.

c) 5-(4-Chlorphenoxy)-pentylmalonsäure-ethylester

Nach der in Beispiel 1c) beschriebenen Arbeitsweise erhält man 202,5 g 5-(4-Chlorphenoxy)-pentylmalonsäureethylester (als zähes Öl) aus 250,6 g 5-(4-Chlorphenoxy)-pentylmalonsäurediethylester und 44,6 g Kaliumhydroxid in 500 ml Ethanol.

d) 5-(4-Chlorphenoxy)-pentylmalonsäure-diethylester

Nach der in Beispiel 2d) beschriebenen Arbeitsweise erhält man 265 g 5-(4-Chlorphenoxy)-pentylmalonsäurediethylester [vom Kp. 160–161° bei 0,01 Torr (1,3 Pa)] aus 212,6 g 5-(4-Chlorphenoxy)-pentylbromid, 185,8 g Malonsäurediethylester und einer Lösung von 17,7 g Natrium in 1,25 l Ethanol.

Beispiel 11

2-(3-Benzyloxypropyl)-oxiran-2-carbonsäure-ethylester

a) 2-(3-Benzyloxypropyl)-oxiran-2-carbonsäureethylester

Nach der in Beispiel 1a) beschriebenen Arbeitsweise erhält man 7,2 g der Titelverbindung [als fast farbloses Öl, gereinigt durch Chromatographie an Kieselgel (Laufmittel: Petrolether/Essigsäureethylester 9:1)] aus 26 g 5-Benzyloxy-2-methylenvaleriansäureethylester und 48 g m-Chlorperbenzoesäure in 200 ml Methylenchlorid.

b) 5-Benzyloxy-2-methylenvaleriansäureethylester

Nach der in Beispiel 1b) beschriebenen Arbeitsweise erhält man 30,1 g 5-Benzyloxy-2-methylenvaleriansäureethylester [als gelbliches Öl, gereinigt durch Chromatographie an Kieselgel (Laufmittel: Petrolether/Essigsäureethylester 9:1)] aus 44 g 3-Benzyloxypropylmalonsäureethylester, 30 ml Pyridin, 2 ml Piperidin und 6,6 g Paraformaldehyd.

c) 3-Benzyloxypropylmalonsäureethylester

Nach der in Beispiel 1c) beschriebenen Arbeitsweise erhält man 44,5 g 3-Benzyloxypropylmalonsäureethylester (als dickes, gelbliches Öl) aus 67 g 3-Benzyloxypropylmalonsäurediethylester und 12,4 g Kaliumhydroxid in 200 ml Ethanol.

d) 3-Benzyloxypropylmalonsäurediethylester

Nach der in Beispiel 2d) beschriebenen Arbeitsweise erhält man 67,4 g 3-Benzyloxypropylmalonsäurediethylester (als gelbliches Öl) aus 86 g 3-Benzyloxypropylbromid, 78 g Malonsäurediethylester und einer Lösung von 9 g Natrium in 600 ml Ethanol.

Beispiel 12

2-[5-(4-Chlorphenoxy)-pentyl]-oxiran-2-carbonsäure

2,0 g 2-[5-(4-Chlorphenoxy)-pentyl]-oxiran-2-carbonsäureethylester, 6,4 ml 1n Natronlauge und 6,4 ml Tetrahydrofuran werden bei Raumtemperatur bis zur Bildung einer klaren Lösung (ca. 1 Stunde) gerührt; die Lösung wird im Vakuum auf die Hälfte des Volumens eingeengt und unter Eiskühlung mit 6,5 ml 1n Salzsäure versetzt. Man extrahiert 3mal mit je 20 ml Diethylether, trocknet die vereinigten organischen Phasen über Natriumsulfat und engt ein. Zurück bleiben 1,65 g der Titelverbindung als zähes Öl.

Beispiel 13
Natrium-2-[4-(3-trifluormethylphenoxy)-butyl]-oxiran-2-carboxylat

4,0 g 2-[4-(3-Trifluormethylphenoxy)-butyl]-oxiran-2-carbonsäureethylester, 12 ml 1n Natronlauge und 16 ml Tetrahydrofuran werden 2 Stunden bei Raumtemperatur gerührt; die Lösung wird auf die Hälfte des Volumens eingeengt und zweimal mit je 50 ml Diethylether extrahiert. Aus der wässerigen Phase kristallisiert beim Stehen ein Niederschlag aus, der abfiltriert und mit wenig Wasser und Diethylether gewaschen wird. Durch sukzessives Einengen der Filtrate erhält man 2,44 g der Titelverbindung vom F. 94–97°.

Beispiel 14
Natrium-2-[4-(3-Chlorphenoxy)-butyl]-oxiran-2-carboxylat

Nach der in Beispiel 13 beschriebenen Arbeitsweise erhält man 11,4 g der Titelverbindung vom F. 102–106° aus 13,1 g 2-[4-(3-Chlorphenoxy)-butyl]-oxiran-2-carbonsäureethylester, 43,8 ml 1n Natronlauge und 50 ml Tetrahydrofuran.

Beispiel 15
Calcium-2-[4-(3-Chlorphenoxy)-butyl]-oxiran-2-carboxylat

1 g Natrium-2-[4-(3-Chlorphenoxy)-butyl]-oxiran-2-carboxylat, gelöst in 10 ml Wasser, werden mit einer Lösung von 400 mg Calciumchlorid in 5 ml Wasser versetzt; es wird vom abgeschiedenen schmierigen Niederschlag abdekantiert, der Rückstand zweimal mit Wasser verrieben und dekantiert. Der Rückstand wird über Phosphorpentoxid getrocknet. Man erhält 840 mg der Titelverbindung, die sich ab 225° ohne zu schmelzen zersetzt.

Beispiel 16
2-[4-(3-Trifluormethylphenoxy)-butyl]-oxiran-2-carbonsäuremethylester

5,4 g der Titelverbindung [farbloses Öl, gereinigt durch Chromatographie an Kieselgel (Laufmittel: Petrolether/Methylenchlorid 1:1)]; $R_f$ 0,5 bei Dünnschichtchromatographie an Kieselgel mit Petrolether/Essigsäureethylether/Essigsäure (80:20:3)] erhält man analog Beispiel 3a) aus 7,8 g 2-Methylen-6-(3-trifluormethylphenoxy)-hexansäuremethylester und 11,0 g Chlorperbenzoesäure.

8,0 g der Ausgangsverbindung 2-Methylen-6-(3-trifluormethylphenoxy)-hexansäuremethylester erhält man aus 17,5 g 4-(3-Trifluormethylphenoxy)-butylmalonsäuredimethylester analog Beispiel 3b) und 3c).

Beispiel 17
2-[2-(3-Phenylpropyloxy)-ethyl]-oxiran-2-carbonsäureethylester

a) 2-[2-(3-Phenylpropyloxy)-ethyl]-oxiran-2-carbonsäureethylester

Nach der in Beispiel 1a) beschriebenen Arbeitsweise erhält man 6,6 g der Titelverbindung als farbloses Öl vom Kp. 142° bei 0,15 Torr (20 Pa) [Reinigung durch Chromatographie an Kiselgel (Laufmittel: Petrolether/Essigsäureethylester 9:1)] aus 18,0 g 2-Methylen-4-(3-phenylpropyloxy)-buttersäureethylester und 22 g m-Chlorperbenzoesäure in 150 ml Methylenchlorid.

b) 2-Methylen-4-(3-phenylpropyloxy)-buttersäureethylester

Nach der in Beispiel 1b) beschriebenen Arbeitsweise erhält man 27 g 2-Methylen-4-(3-phenylpropyloxy)-buttersäureethylester als farbloses Öl vom Kp. 140° bei 0,1 Torr (13 Pa) aus 45 g 2-(3-Phenylpropyloxy)-ethylmalonsäureethylester, 5,74 g Paraformaldehyd, 28,5 ml Pyridin und 1,9 ml Piperidin.

c) 2-(3-Phenylpropyloxy)-ethylmalonsäureethylester

Nach der in Beispiel 1c) beschriebenen Arbeitsweise erhält man 46,4 g 2-(3-Phenylpropyloxy)-ethylmalonsäureethylester als gelbliches Öl aus 54 g 2-(3-Phenylpropyloxy)-ethylmalonsäurediethylester und 10,68 g Kaliumhydroxyd in 120 ml Ethanol.

d) 2-(3-Phenylpropyloxy)-ethylmalonsäurediethylester

Nach der in Beispiel 2d) beschriebenen Arbeitsweise erhält man 54 g 2-(3-Phenylpropyloxy)-ethylmalonsäurediethylester vom Kp. 168–171° bei 0,2 Torr (27 Pa) aus 72,7 g 2-(3-Phenylpropyloxy)-ethylchlorid, 87,9 g Malonsäurediethylester, 3 g Kaliumiodid und einer Lösung von 12,6 g Natrium in 300 ml Ethanol.

Beispiel 18
2-[2-(4-Chlorbenzyloxy)-ethyl]-oxiran-2-carbonsäureethylester

a) 2-[2-(4-Chlorbenzyloxy)-ethyl]-oxiran-2-carbonsäureethylester

Nach der in Beispiel 1a) beschriebenen Arbeitsweise erhält man 6,2 g der Titelverbindung vom Kp. 130–135° bei 0,05 Torr (7 Pa) aus 10,3 g 4-(4-Chlorbenzyloxy)-2-methylenbuttersäureethylester und 13 g m-Chlorperbenzoesäure in 100 ml Methylenchlorid.

b) 4-(4-Chlorbenzyloxy)-2-methylenbuttersäureethylester

Nach der in Beispiel 1b) beschriebenen Arbeitsweise erhält man 14 g 4-(4-Chlorbenzyloxy)-2-methylenbuttersäureethylester vom Kp. 125–130° bei 0,1 Torr (13,3 Pa) aus 19,0 g 2-(4-Chlorbenzyloxy)-ethylmalonsäureethylester, 2,0 g Paraformaldehyd, 12 ml Pyridin und 0,8 ml Piperidin.

c) 2-(4-Chlorbenzyloxy)-ethylmalonsäureethylester

Nach der in Beispiel 1c) beschriebenen Arbeitsweise erhält man 19,5 g 2-(4-Chlorbenzyloxy)-ethylmalonsäureethylester aus 26,0 g 2-(4-Chlorbenzyloxy)-ethylmalonsäurediethylester und 5,05 g Kaliumhydroxid in 100 ml Ethanol.

d) 2-(4-Chlorbenzyloxy)-ethylmalonsäure-diethylester

Nach der in Beispiel 2d) beschriebenen Arbeitsweise erhält man 60,3 g 2-(4-Chlorbenzyloxy)-ethylmalonsäurediethylester vom Kp. 168° bei 0,2 Torr (27 Pa) aus 87 g 2-(4-Chlorbenzyloxy)-ethylchlorid, 102 g Malonsäurediethylester, einer Lösung aus 14,6 g Natrium in 300 ml Ethanol und 3 g Kaliumiodid.

Beispiel 19

2-[4-(4-Methylphenoxy)-butyl]-oxiran-2-carbonsäureethylester

Alternativ zu Beispiel 2 wird die Titelverbindung wie folgt erhalten: Man gibt 17,6 g frisch zerriebenes Maleinsäureanhydrid zu einer bei 0° hergestellten Lösung von 6,0 g 85%igem Wasserstoffperoxid in 100 ml Methylenchlorid auf einmal zu, wobei die Temperatur von 0 auf −5° sinkt. Nach einstündigem Rühren im Eisbad versetzt man die Lösung von Permaleinsäure mit 17,0 g 6-(4-Methylphenoxy)-2-methylenhexansäureethylester in 50 ml Methylenchlorid und erhitzt anschliessend 16 Stunden unter Rückfluss zum Sieden.

Zur Aufarbeitung filtriert man von der abgeschiedenen Maleinsäure ab, rührt zwei Stunden mit 150 ml gesättigter Natriumhydrogencarbonatlösung zuerst unter Eiskühlung, dann bei Raumtemperatur, trennt die organische Phase ab und wiederholt das Ausrühren einmal. Nach erneuter Phasentrennung wird die organische Phase mit 30 g festem Natriumhydrogensulfit gerührt (1 Stunde), (Peroxidnachweis negativ), filtriert, vollständig eingeengt und der Rückstand destilliert. Man erhält 10,5 g der Titelverbindung als hellgelbes Öl, das durch Chromatographie an einer Kieselgelsäule (Laufmittel: Petrolether/Methylenchlorid 1:1) gereinigt wird. $R_f$: 0,4 bei Dünnschichtchromatographie an einer Kieselgelplatte mit Chloroform.

Galenische Beispiele:

Beispiel 1

Ansatz für Ampullen

100 g 2-[5-(4-Chlorphenoxy)-pentyl]-oxiran-2-carbonsäure werden in ca. 8 Liter aqua bidest. unter Zusatz der äquivalenten Menge Natronlauge gelöst. Die Lösung wird auf pH 7,0 ± 0,5 eingestellt und mit aqua bidest. auf 10 Liter aufgefüllt. Dann wird steril filtriert und unter keimfreien Bedingungen in 2 ml-Ampullen abgefüllt.

Beispiel 2

10 000 Kapseln mit einem Wirkstoffgehalt von 30 mg werden aus folgenden Bestandteilen hergestellt:

300 g 2-(5-Phenoxypentyl)-oxiran-2-carbonsäureethylester werden mit 500 g Neutralöl gemischt und in Weichgelatinekapseln abgefüllt.

Beispiel 3

1000 Kapseln mit einem Wirkstoffgehalt von 25 mg werden wie folgt hergestellt:

25 g 2-[4-(3-Chlorphenoxy)-butyl]-oxiran-2-carbonsäureethylester werden in 100 ml Methylenchlorid gelöst. Die Lösung wird mit 75 g mikronisierter Kieselsäure gut gemischt. Die Mischung wird zur Trockne eingedampft und dann in Hartgelatinekapseln abgefüllt.

Beispiel 4

10 000 Kapseln mit einem Wirkstoffgehalt von 20 mg werden wie folgt hergestellt:

200 g 2-[4-(3-Trifluormethylphenoxy)-butyl]-oxiran-2-carbonsäureethylester werden in 1000 ml Methylenchlorid gelöst. Die Lösung wird mit 800 g mikronisierter Kieselsäure gut gemischt. Die Mischung wird zur Trockne eingedampft und dann in Hartgelatinekapseln abgefüllt.

Beispiel 5

10 000 Kapseln mit einem Wirkstoffgehalt von 25 mg werden wie folgt hergestellt:

250 g 2-[6-(4-Chlorphenoxy)-hexyl]-oxiran-2-carbonsäureethylester werden in 1000 ml Methylenchlorid gelöst. Die Lösung wird mit 750 g mikronisierter Kieselsäure gut gemischt. Die Mischung wird zur Trockne eingedampft und dann in Hartgelatinekapseln abgefüllt.

Beispiel 6

Tabletten mit einem Wirkstoffgehalt von 25 mg werden wie folgt hergestellt:

1,0 kg Natrium-2-[4-(3-trifluormethylphenoxy)-butyl]-oxiran-2-carboxylat, 4,5 kg Xylit und 3,0 kg Calciumphosphat werden mit 0,25 kg Polyvinylpyrrolidon (MG 25 000; MG = Molekulargewicht) in ungefähr 0,5 l Wasser granuliert. Das Granulat wird durch ein Sieb von 1,25 mm Maschenweite gesiebt und nach dem Trocknen werden 0,9 kg Carboxymethylcellulose, 0,25 kg Talkum und 0,1 kg Magnesiumstearat zugegeben. Man verpresst das trockene Granulat zu Tabletten von 8 mm Durchmesser, 250 mg Gewicht und einer Härte von 5–6 kg.

Beispiel 7

10 000 Kapseln mit einem Wirkstoffgehalt von 20 mg werden wie folgt hergestellt:

200 g 2-[5-(4-Chlorphenoxy)-pentyl]-oxiran-2-carbonsäureethylester werden in 1000 ml Methylenchlorid gelöst. Die Lösung wird mit 800 g mikronisierter Kieselsäure gut gemischt. Die Mischung wird zur Trockne eingedampft und dann in Hartgelatinekapseln abgefüllt.

Die folgenden Beispiele beschreiben die Herstellung von Vorprodukten, die analog Genzer et al. umgesetzt werden.

Beispiel A

a) 2-[3-(5-Chlor-2-methoxyphenyl)-propyloxy]-ethylchlorid

11,2 g 2-[3-(5-Chlor-2-methoxyphenyl)-propyloxy]-ethanol werden mit 20 ml Thionylchlorid bei 50° 3 Stunden gerührt. Anschliessend wird das überschüssige Thionylchlorid im Vakuum abdestilliert. Zurück bleiben 11,3 g 2-[3-(5-Chlor-2-methoxyphenyl)-propyloxy]-ethylchlorid als braunes Öl.

b) 2-[3-(5-Chlor-2-methoxyphenyl)-propyloxy]-ethanol

Zu einer Lösung von 1,6 g Natrium in 20 g Ethylenglykol werden 10 ml Xylol gegeben und anschliessend eine Lösung von 21,9 g 3-(5-Chlor-2-methoxyphenyl)-propylchlorid in 10 ml Xylol zugetropft. Man kocht 1 Stunde unter Rückfluss, lässt abkühlen, versetzt mit 100 ml Wasser und extrahiert 3mal mit je 100 ml Diethylether. Die vereinigten organischen Phasen werden über Natriumsulfat getrocknet und eingeengt. Zurück bleiben 11,2 g 2-[3-(5-Chlor-2-methoxyphenyl)-propyloxy]-ethanol als braunes Öl.

c) 3-(5-Chlor-2-methoxyphenyl)-propylchlorid

Es werden 58,2 g 3-(5-Chlor-2-methoxyphenyl)-propanol-1 und 50 ml Thionylchlorid 8 Stunden bei 50° gerührt, das überschüssige Thionylchlorid im Vakuum abdestilliert und der Rückstand im Hochvakuum destilliert. Man erhält 50,9 g 3-(5-Chlor-2-methoxyphenyl)-propylchlorid vom Kp. 87–95° bei 0,005 Torr (0,66 Pa).

d) 3-(5-Chlor-2-methoxyphenyl)-propanol-1

Nach der in Beispiel Bd) beschriebenen Arbeitsweise erhält man aus 96,6 g 3-(5-Chlor-2-methoxyphenyl)-propionsäure und 14 g Lithiumaluminiumhydrid in 900 ml Diethylether 66,7 g 3-(5-Chlor-2-methoxyphenyl)-propanol-1 vom Kp. 94–97° bei 0,001 Torr (0,13 Pa).

e) 3-(5-Chlor-2-methoxyphenyl)-propionsäure

Nach der in Beispiel Be) beschriebenen Arbeitsweise erhält man aus 100 g 5-Chlor-2-methoxybenzylchlorid, 120 ml Malonsäurediethylester und einer Lösung von 12,07 g Natrium in 1,1 l Ethanol 124 g 5-Chlor-2-methoxybenzylmalonsäurediethylester, der nach Verseifung mit Kaliumhydroxid und Erhitzen der entstandenen 5-Chlor-2-methoxybenzylmalonsäure auf 160–170° 63,2 g 3-(5-Chlor-2-methoxyphenyl)-propionsäure F. 91–92° ergibt.

Beispiel B

a) 3-[3-(4-Fluorphenyl)-propyloxy]-propylchlorid

Nach der in Beispiel Aa) beschriebenen Arbeitsweise erhält man aus 9,9 g 3-[3-(4-Fluorphenyl)-propyloxy]-propanol-1 und 20 ml Thionylchlorid 10,0 g der Titelverbindung als braunes Öl.

b) 3-[3-(4-Fluorphenyl)-propyloxy]-propanol-1

Nach der in Beispiel Ab) beschriebenen Arbeitsweise erhält man aus einer Lösung von 1,6 g Natrium in 25 g 1,3-Propandiol, 20 ml Xylol und 17,2 g 3-(4-Fluorphenyl)-propylchlorid 9,9 g der Titelverbindung als braunes Öl.

c) 3-(4-Fluorphenyl)-propylchlorid

Nach der in Beispiel Ac) beschriebenen Arbeitsweise erhält man 30,2 g 3-(4-Fluorphenyl)-propylchlorid (Öl) aus 35 g 3-(4-Fluorphenyl)-propanol-1 und 30 ml Thionylchlorid.

d) 3-(4-Fluorphenyl)-propanol-1

Zu einer Suspension von 19,7 g Lithiumaluminiumhydrid in 300 ml Tetrahydrofuran tropft man bei ca. 45 °C Reaktionstemperatur unter Rühren eine Lösung von 43,6 g 3-(4-Fluorphenyl)-propionsäure in 300 ml Tetrahydrofuran. Nach beendeter Zugabe hält man noch 2,5 Stunden bei dieser Temperatur, tropft dann nacheinander 80 ml Wasser und 20 ml 4n Natronlauge zu. Man filtriert den Niederschlag ab, wäscht mehrmals mit Diethylether nach, trocknet die vereinigten Lösungen über Natriumsulfat und engt ein. Zurück bleiben 39,7 g 3-(4-Fluorphenyl)-propanol-1 als fast farbloses Öl.

e) 3-(4-Fluorphenyl)-propionsäure

Zu einer Lösung von 12,6 g Natrium in 300 ml Ethanol tropft man 91,6 g Malonsäurediethylester, rührt noch 15 Minuten nach und tropft dann 98,3 g 4-Fluorbenzylbromid zu. Man kocht anschliessend noch 3 Stunden unter Rückfluss, destilliert den grössten Teil des Lösungsmittels ab, nimmt den Rückstand mit Eiswasser (800 ml) und Methylenchlorid (600 ml) auf und schüttelt gut durch. Die organische Phase wird gesammelt, eingeengt und das zurückbleibende Öl (4-Fluorbenzylmalonsäurediethylester) (137,6 g) mit einer Lösung von 133 g Kaliumhydroxid in 780 ml Methanol 12 Stunden gerührt. Man engt im Vakuum weitgehend ein, löst den Rückstand in Wasser/Diethylether, schüttelt gut durch, trennt die organische Phase ab und säuert die wässrige Phase unter Eiskühlung mit 10n Schwefelsäure an. Man extrahiert mit Methylenchlorid, engt die organische Phase ein und verrührt den öligen Rückstand mit Petrolether/Essigester (3:1), wobei 53,6 g 4-Fluorbenzylmalonsäure auskristallisieren (F. 134–136 °C).

Die 4-Fluorbenzylmalonsäure wird 1,5 Stunden lang auf 170–175° erhitzt. Das Reaktionsprodukt wird nach dem Abkühlen mit wenig Diethylether verrührt. Hierbei kristallisieren 41,6 g 3-(4-Fluorphenyl)-propionsäure vom F. 85–88° [aus Essigester/Petrolether (1:4)] aus.

Beispiel C

a) 3-(3-Trifluormethylphenyl)-propylchlorid

Nach der in Beispiel Aa) beschriebenen Arbeitsweise erhält man 10,3 g 3-(3-Trifluormethylphenyl)-propylchlorid (Öl) aus 12 g 3-(3-Trifluormethylphenyl)-propanol-1 und 10 g Thionylchlorid.

b) 3-(3-Trifluormethylphenyl)-propanol-1

Man tropft bei 0–10 °C eine Lösung von 57 g Oxiran in 120 ml Diethylether zu einer Grignard-Lösung, hergestellt aus 14,8 g Magnesium und 100 g 3-(Chlormethyl)-benzotrifluorid in 450 ml Diethylether. Man rührt 1 Stunde bei Raumtemperatur nach und zersetzt anschliessend mit 300 ml 10%iger Schwefelsäure unter Eiskühlung. Man sammelt die organische Phase, extrahiert noch 2 mal mit Diethylether, trocknet die vereinigten organischen Phasen über Magnesiumsulfat und destilliert. Man erhält 76,7 g 3-(3-Trifluormethylphenyl)-propanol-1 vom Kp. 85–95 °C bei 0,02 Torr (2,66 Pa).

Pharmakologie

Die erfindungsgemässen Phen(alk)oxy-substituierten Oxirancarbonsäuren der allgemeinen Formel I senken den Blutglucosespiegel und den Blutspiegel der Ketonkörper, wobei sie sich in ihrer chemischen Struktur und ihrer Wirksamkeit grundlegend von pankreaswirksamen, betacytotropen Substanzen (z.B. Sulfonylharnstoffen) durch ihre extrapankreatische Wirkung unterscheiden und extrapankreatisch wirkenden Handelspräparaten (z.B. Buformin und Phenformin) überlegen erweisen.

In der anschliessenden Tabelle werden die untersuchten Verbindungen durch eine laufende Nummer gekennzeichnet, die wie folgt zuzuordnen ist:

| lfd. Nr. | Name der Verbindung |
|---|---|
| 1 | Buformin |
| 2 | Phenformin |
| 3 | 2-[4-(3-Chlorphenoxy)-butyl]-oxiran-2-carbonsäureethylester |
| 4 | 2-[4-(3-Trifluormethylphenoxy)-butyl]-oxiran-2-carbonsäureethylester |
| 5 | 2-[6-(4-Chlorphenoxy)-hexyl]-oxiran-2-carbonsäureethylester |
| 6 | 2-[5-(4-Chlorphenoxy)-pentyl]-oxiran-2-carbonsäureethylester |
| 7 | 2-[4-(4-Methylphenoxy)-butyl]-oxiran-2-carbonsäureethylester |
| 8 | 2-(4-Phenoxybutyl)-oxiran-2-carbonsäureethylester |
| 9 | 2-(5-Phenoxypentyl)-oxiran-2-carbonsäureethylester |

In Tabelle I werden Untersuchungen des Einflusses von Vertretern der erfindungsgemässen Verbindungen auf die Blutglucosekonzentration nüchterner stoffwechselgesunder Ratten nach einmaliger oraler Substanzgabe von 0,03–0,36 mmol/kg Körpergewicht innerhalb von 6 Stunden wiedergegeben. In Spalte A ist die Wirkstoffdosis (mg/kg) angegeben, die bei 50% der Tiere eine Senkung der Blutglucosekonzentration um mindestens 25% im Vergleich zur Kontrollgruppe bewirkt, in Spalte B diejenige, die bei 50% der Tiere eine Senkung der Blutglucosekonzentration um mindestens 15% im Vergleich zur Kontrollgruppe bewirkt.

In der Spalte C werden Daten zur akuten Toxizität ($LD_{50}$ Maus p.o.) wiedergegeben.

Tabelle I

| Lfd. Nr. | A $ED_{50}$(25%) [mg/kg] Ratte p.o. | B $ED_{50}$(15%) [mg/kg] Ratte p.o. | C Toxizität $LD_{50}$ [mg/kg] Maus p.o. |
|---|---|---|---|
| 1 | 194 | >100 | 475 |
| 2 | >343 | >150 | 410* |
| 3 | 24 | 3 | 350 |

Tabelle I (Fortsetzung)

| Lfd. Nr. | A $ED_{50}$(25%) [mg/kg] Ratte p.o. | B $ED_{50}$(15%) [mg/kg] Ratte p.o. | C Toxizität $LD_{50}$ [mg/kg] Maus p.o. |
|---|---|---|---|
| 4 | 13 | ~ 7 | 460 |
| 5 | 7 | < 7 | 300 |
| 6 | 6 | 5 | 310 |
| 7 | 19 | 8 | 460 |
| 8 | 87 | 37 | – |
| 9 | < 18 | 8 | 370 |

* Zit. Blickens, D. A; Riggi, S.J.: Toxicol. Appl. Pharmacol. 14(1969) 393–400

Zu Tabelle I:

Spalte A = Dosis, die eine Senkung der Blutglucosekonzentration um 25% bei 50% der Tiere bewirkt

Spalte B = Dosis, die eine Senkung der Blutglucosekonzentration um 15% bei 50% der Tiere bewirkt

Spalte C = Akute Toxizität ($LD_{50}$ in mg/kg; Maus p.o.).

Die Ermittlung der pharmakologischen Eigenschaften erfolgte nach folgenden Methoden:

1. Blutglucosebestimmung nach einmaliger oraler Applikation

Zur Verwendung kommen junge männliche Sprague-Dawley-Ratten (Körpergewicht 150–200 g). Die Haltung der Tiere (12 Tiere pro Dosis) erfolgt in Makrolon-Käfigen mit bis zu 4 Tieren pro Käfig (Raumtemperatur 23 °C, rel. Luftfeuchtigkeit 55%, fester Tag/Nacht-Rhythmus (12/12 h), Standarddiät Altromin®). Den Ratten wird 18 Stunden vor der 1. Blutentnahme das Futter entzogen. Wasseraufnahme erfolgt ad libitum. Blutentnahmen erfolgen unmittelbar vor und 3 und 6 Stunden nach Substanzgabe durch Punktion aus dem retroorbitalen Plexus.

Nach Enteiweissung mit Perchlorsäure erfolgt die Blutglucosebestimmung mittels der enzymatischen HK/G-6-PDH-Methode nach R. Richterich (Klinische Chemie, Theorie und Praxis, 3. Aufl. 1971, S. Karger Verlag, Zürich-Basel, Seite 275). Zum Vergleich wird jeweils eine mit reinem Lösungsmittel behandelte Kontrollgruppe mituntersucht. Die effektiven Dosen (ED-Werte) werden aus der Dosis-Wirkungs-Beziehung durch lineare oder loglineare Regression errechnet.

2. Bestimmung der Toxizität

Die Toxizitätsuntersuchungen werden an weiblichen NMRI-Mäusen (Körpergewicht 22–26 g) durchgeführt. Die Tiere (5 Tiere pro Dosis) erhalten 18 Stunden vor der Behandlung das Futter (Altomin®) auf 50 g/50 Tiere reduziert und Wasser ad libitum. Verschiedene Dosen der Substanzen werden oral mittels Schlundsonde verabreicht (Volumen 10 ml/kg). Die Beobachtungsdauer beträgt 7 Tage. Die $LD_{50}$, d.h. die Dosis, bei der 50%

der Tiere sterben, wird aus der Dosiswirkungskurve graphisch ermittelt.

**Patentansprüche für die Vertragsstaaten: BE CH DE FR GB IT LI LU NL SE**

1. Phen(alk)oxy-substituierte Oxirancarbonsäuren der allgemeinen Formel I

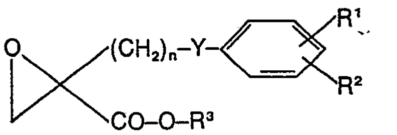
(I),

worin

$R^1$ ein Wasserstoffatom, ein Halogenatom, eine C1–C4-Niederalkylgruppe, eine C1–C4-Niederalkoxygruppe, eine Nitrogruppe oder eine Trifluormethylgruppe bedeutet,

$R^2$ eine der Bedeutungen von $R^1$ hat,

$R^3$ ein Wasserstoffatom oder eine C1–C4-Niederalkylgruppe,

Y die Gruppierung $-O-(CH_2)_m-$,

m 0 oder eine ganze Zahl von 1 bis 4 und

n eine ganze Zahl von 2 bis 8 bedeuten,

wobei die Summe von m und n eine ganze Zahl von 2 bis 8 ist, und die Salze der Carbonsäuren.

2. Phen(alk)oxy-substituierte Oxirancarbonsäuren der allgemeinen Formel I*

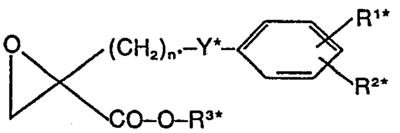
(I*),

worin

$R^{1*}$ und $R^{2*}$ meta- oder para-ständig sind und

$R^{1*}$ ein Wasserstoffatom, ein Chloratom, eine Methylgruppe, eine Methoxygruppe, eine Nitrogruppe oder eine Trifluormethylgruppe,

$R^{2*}$ ein Wasserstoffatom oder ein Chloratom,

$R^{3*}$ ein Wasserstoffatom oder eine C1–C4-Niedrigalkylgruppe,

Y* die Gruppierung $-O-(CH_2)_{m^*}-$,

m* 0 oder 1 und

n* eine ganze Zahl von 3 bis 7 bedeuten,

wobei die Summe von m* und n* eine ganze Zahl von 3 bis 7 ist, und die Salze der Carbonsäuren.

3. Phenoxy-substituierte Oxirancarbonsäuren der allgemeinen Formel

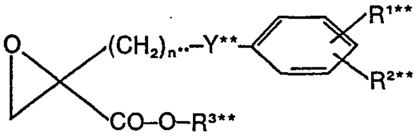
(I**),

worin

$R^{1**}$ meta- oder para-ständig ist und

$R^{1**}$ ein Wasserstoffatom, ein Chloratom oder eine Trifluormethylgruppe,

$R^{2**}$ ein Wasserstoffatom,

$R^{3**}$ ein Wasserstoffatom, eine Methylgruppe oder eine Ethylgruppe,

Y** die Gruppierung –O–,

n** eine ganze Zahl von 4 bis 6 bedeuten,

und die pharmakologisch verträglichen Salze der Carbonsäuren.

4. Verbindung nach Anspruch 3, ausgewählt aus der Gruppe 2-[4-(3-Chlorphenoxy)-butyl]-oxiran-2-carbonsäure-ethylester, 2-[4-(3-Trifluormethylphenoxy)-butyl]-oxiran-2-carbonsäureethylester, 2-[6-(4-Chlorphenoxy)-hexyl]-oxiran-2-carbonsäureethylester, 2-[5-(4-Chlorphenoxy)-pentyl]-oxiran-2-carbonsäureethylester, den entsprechenden Oxiran-2-carbonsäuren sowie deren pharmakologisch verträglichen Salzen.

5. Verbindung nach Anspruch 3, worin in Formel I** $R^{1**}$ ein paraständiges Chloratom, $R^{2**}$ ein Wasserstoffatom, $R^{3**}$ eine Ethylgruppe, Y** ein Sauerstoffatom und n** 6 bedeuten.

6. Arzneimittel, enthaltend eine oder mehrere Verbindungen nach einem der Ansprüche 1 bis 5.

7. Phen(alk)oxy-substituierte Oxirancarbonsäuren der allgemeinen Formel I, worin die Substituenten die in Anspruch 1 angegebene Bedeutung haben, zur Anwendung bei der Behandlung und Prophylaxe von Krankheiten, die auf Störungen des Glucose- und Fettstoffwechsels beruhen.

8. Verfahren zur Herstellung von Phen(alk)oxy-substituierten Oxirancarbonsäuren der allgemeinen Formel I

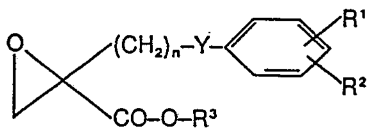
(I),

worin

$R^1$ ein Wasserstoffatom, ein Halogenatom, eine C1–C4-Niederalkylgruppe, eine C1–C4-Niederalkoxygruppe, eine Nitrogruppe oder eine Trifluormethylgruppe bedeutet,

$R^2$ eine der Bedeutungen von $R^1$ hat,

$R^3$ ein Wasserstoffatom oder eine C1–C4-Niederalkylgruppe,

Y die Gruppierung $-O-(CH_2)_m-$,

m 0 oder eine ganze Zahl von 1 bis 4 und

n eine ganze Zahl von 2 bis 8 bedeuten,

wobei die Summe von m und n eine ganze Zahl von 2 bis 8 ist, und den Salzen der Säuren, dadurch gekennzeichnet, dass man eine substituierte α-Methylencarbonsäure der allgemeinen Formel II

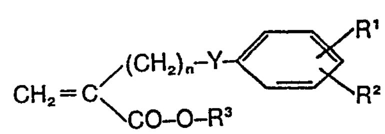
(II),

worin $R^1$, $R^2$, $R^3$, Y und n die vorstehend angegebene Bedeutung haben, oxidiert und gegebenenfalls anschliessend die erhaltenen Niederalkylester verseift oder die erhaltenen Säuren in die Salze oder Niederalkylester überführt.

9. Verfahren zur Herstellung von Phen(alk)oxy-substituierten Oxirancarbonsäuren der allgemeinen Formel I*

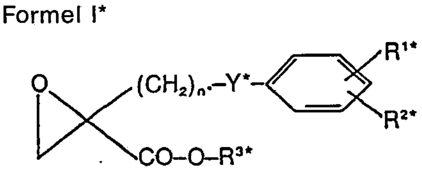
(I*),

worin

$R^{1*}$ und $R^{2*}$ meta- oder para-ständig sind und

$R^{1*}$ ein Wasserstoffatom, ein Chloratom, eine Methylgruppe, eine Methoxygruppe, eine Nitrogruppe oder eine Trifluormethylgruppe,

$R^{2*}$ ein Wasserstoffatom oder ein Chloratom,

$R^{3*}$ ein Wasserstoffatom oder eine C1–C4-Niedrigalkylgruppe,

$Y^*$ die Gruppierung $-O-(CH_2)_{m^*}-$,

$m^*$ 0 oder 1 und

$n^*$ eine ganze Zahl von 3 bis 7 bedeuten,

wobei die Summe von $m^*$ und $n^*$ eine ganze Zahl von 3 bis 7 ist, und den Salzen der Säuren, dadurch gekennzeichnet, dass man eine substituierte α-Methylencarbonsäure der allgemeinen Formel II*

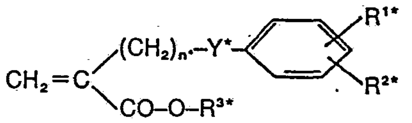

(II*),

worin $R^{1*}$, $R^{2*}$, $R^{3*}$, $Y^*$ und $n^*$ die vorstehend angegebene Bedeutung haben, oxidiert und gegebenenfalls anschliessend die erhaltenen Niederalkylester verseift oder die erhaltenen Säuren in die Salze oder Niederalkylester überführt.

10. Verfahren zur Herstellung von Phenoxysubstituierten Oxirancarbonsäuren der allgemeinen Formel I**

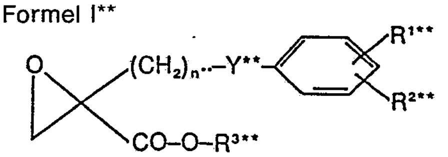

(I**),

worin

$R^{1**}$ meta- oder para-ständig ist und

$R^{1**}$ ein Wasserstoffatom, ein Chloratom oder eine Trifluormethylgruppe,

$R^{2**}$ ein Wasserstoffatom,

$R^{3**}$ ein Wasserstoffatom, eine Methylgruppe oder eine Ethylgruppe,

$Y^{**}$ die Gruppierung $-O-$,

$n^{**}$ eine ganze Zahl von 4 bis 6 bedeuten,

und den Salzen der Säuren, dadurch gekennzeichnet, dass man eine substituierte α-Methylencarbonsäure der allgemeinen Formel II**

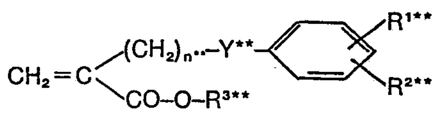

(II**),

worin $R^{1**}$, $R^{2**}$, $R^{3**}$, $Y^{**}$ und $n^{**}$ die vorstehend angegebene Bedeutung haben, oxidiert und gegebenenfalls anschliessend die erhaltenen Niederalkylester verseift oder die erhaltenen Säuren in die Salze oder Niederalkylester überführt.

**Patentansprüche für den Vertragsstaat: AT**

1. Verfahren zur Herstellung von Phen(alk)oxy-substituierten Oxirancarbonsäuren der allgemeinen Formel I

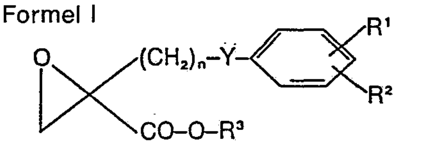

(I),

worin

$R^1$ ein Wasserstoffatom, ein Halogenatom, eine C1–C4-Niederalkylgruppe, eine C1–C4-Niederalkoxygruppe, eine Nitrogruppe oder eine Trifluormethylgruppe bedeutet,

$R^2$ eine der Bedeutungen von $R^1$ hat,

$R^3$ ein Wasserstoffatom oder eine C1–C4-Niederalkylgruppe,

Y die Gruppierung $-O-(CH_2)_m-$,

m 0 oder eine ganze Zahl von 1 bis 4 und

n eine ganze Zahl von 2 bis 8 bedeuten,

wobei die Summe von m und n eine ganze Zahl von 2 bis 8 ist, und den Salzen der Säuren, dadurch gekennzeichnet, dass man eine substituierte α-Methylencarbonsäure der allgemeinen Formel II

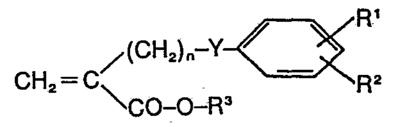

(II),

worin $R^1$, $R^2$, $R^3$, Y und n die vorstehend angegebene Bedeutung haben, oxidiert und gegebenenfalls anschliessend die erhaltenen Niederalkylester verseift oder die erhaltenen Säuren in die Salze oder Niederalkylester überführt.

2. Verfahren zur Herstellung von Phen(alk)oxy-substituierten Oxirancarbonsäuren der allgemeinen Formel I*

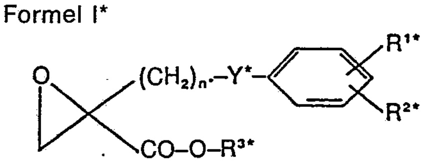

(I*),

worin

$R^{1*}$ und $R^{2*}$ meta- oder para-ständig sind und

$R^{1*}$ ein Wasserstoffatom, ein Chloratom, eine Methylgruppe, eine Methoxygruppe, eine Nitrogruppe oder eine Trifluormethylgruppe,

$R^{2*}$ ein Wasserstoffatom oder ein Chloratom,

$R^{3*}$ ein Wasserstoffatom oder eine C1–C4-Niederalkylgruppe,

$Y^*$ die Gruppierung $-O-(CH_2)_{m^*}-$,

$m^*$ 0 oder 1 und

$n^*$ eine ganze Zahl von 3 bis 7 bedeuten,

wobei die Summe von $m^*$ und $n^*$ eine ganze Zahl von 3 bis 7 ist, und den Salzen der Säuren, dadurch gekennzeichnet, dass man eine substituierte α-Methylencarbonsäure der allgemeinen Formel II*

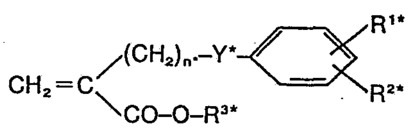

(II*),

worin $R^{1*}$, $R^{2*}$, $R^{3*}$, $Y^*$ und $n^*$ die vorstehend angegebene Bedeutung haben, oxidiert und gegebenenfalls anschliessend die erhaltenen Niederalkylester verseift oder die erhaltenen Säuren in die Salze oder Niederalkylester überführt.

3. Verfahren zur Herstellung von Phenoxysubstituierten Oxirancarbonsäuren der allgemeinen Formel I**

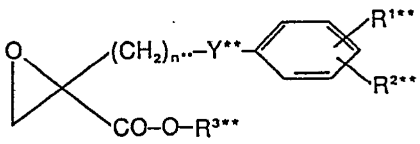
(I**),

worin

$R^{1**}$ meta- oder para-ständig ist und

$R^{1**}$ ein Wasserstoffatom, ein Chloratom oder eine Trifluormethylgruppe,

$R^{2**}$ ein Wasserstoffatom,

$R^{3**}$ ein Wasserstoffatom, eine Methylgruppe oder eine Ethylgruppe,

$Y^{**}$ die Gruppierung –O–,

$n^{**}$ eine ganze Zahl von 4 bis 6 bedeuten,

und den Salzen der Säuren, dadurch gekennzeichnet, dass man eine substituierte α-Methylencarbonsäure der allgemeinen Formel II**

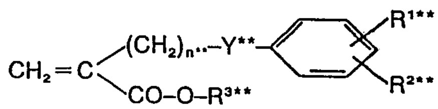
(II**),

worin $R^{1**}$, $R^{2**}$, $R^{3**}$, $Y^{**}$ und $n^{**}$ die vorstehend angegebene Bedeutung haben, oxidiert und gegebenenfalls anschliessend die erhaltenen Niederalkylester verseift oder die erhaltenen Säuren in die Salze oder Niederalkylester überführt.

4. Verfahren nach Anspruch 3, dadurch gekennzeichnet, dass man eine Verbindung der Formel I**, ausgewählt aus der Gruppe 2-[4-(3-Chlorphenoxy)-butyl]-oxiran-2-carbonsäureethylester, 2-[4-(3-Trifluormethylphenoxy)-butyl]-oxiran-2-carbonsäureethylester, 2-[6-(4-Chlorphenoxy)-hexyl]-oxiran-2-carbonsäureethylester, 2-[5-(4-Chlorphenoxy)-pentyl]-oxiran-2-carbonsäureethylester, den entsprechenden Oxirancarbonsäuren sowie deren pharmakologisch verträglichen Salzen herstellt.

5. Verfahren nach Anspruch 3, dadurch gekennzeichnet, dass man die Verbindung der Formel I**, worin $R^{1**}$ ein para-ständiges Chloratom, $R^{2**}$ ein Wasserstoffatom, $R^{3**}$ eine Ethylgruppe, $Y^{**}$ ein Sauerstoffatom und $n^{**}$ 6 bedeuten, herstellt.

6. Verfahren zur Herstellung von hypoglycämisch und hypoketonämisch wirksamen Mitteln, dadurch gekennzeichnet, dass man Phenyl(alk)-oxy-substituierte Oxirancarbonsäuren der allgemeinen Formel I

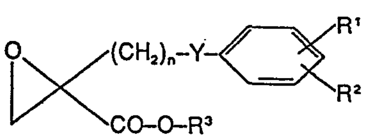
(I),

worin

$R^1$ ein Wasserstoffatom, ein Halogenatom, eine C1–C4-Niederalkylgruppe, eine C1–C4-Niederalkoxygruppe, eine Nitrogruppe oder eine Trifluormethylgruppe bedeutet,

$R^2$ eine der Bedeutungen von $R^1$ hat,

$R^3$ ein Wasserstoffatom oder eine C1–C4-Niederalkylgruppe,

Y die Gruppierung $-O-(CH_2)_m-$,

m 0 oder eine ganze Zahl von 1 bis 4 und

n eine ganze Zahl von 2 bis 8 bedeuten,

wobei die Summe von m und n eine ganze Zahl von 2 bis 8 ist, oder deren pharmakologisch verträgliche Salze herstellt, indem man eine substituierte α-Methylencarbonsäure der allgemeinen Formel II

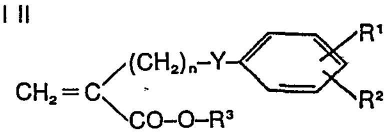
(II),

worin $R^1$, $R^2$, $R^3$, Y und n die vorstehend angegebene Bedeutung haben, oxidiert und gegebenenfalls anschliessend die erhaltenen Niederalkylester verseift oder die erhaltenen Säuren in die Salze oder Niederalkylester überführt, und dass man eine der so erhaltenen Verbindungen durch Mischen mit einem geeigneten pharmazeutisch annehmbaren Träger in eine zur Verabreichung als hypoglycämisch und hypoketonämisch wirksames Mittel geeignete Form bringt.

**Claims for the contracting states: BE, CH, LI, DE, FR, GB, IT, LU, NL, SE:**

1. Phen(alk)oxy-substituted oxiranecarboxylic acids of the general formula I

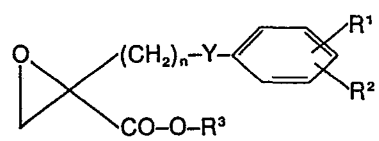
(I)

wherein

$R^1$ denotes a hydrogen atom, a halogen atom, a C1–C4-lower alkyl group, a C1–C4-lower alkoxy group, a nitro group or a trifluoromethyl group,

$R^2$ has one of the meanings of $R^1$,

$R^3$ denotes a hydrogen atom or a C1–C4-lower alkyl group,

Y denotes the grouping $-O-(CH_2)_m-$,

m denotes 0 or an integer from 1 to 4 and

n denotes an integer from 2 to 8, the sum of m and n being an integer from 2 to 8,

and the salts of the carboxylic acids.

2. Phen(alk)oxy-substituted oxiranecarboxylic acids of the general formula I*

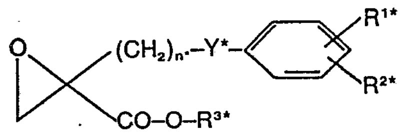
(I*)

wherein

$R^{1*}$ and $R^{2*}$ are in the meta- or para-position and

$R^{1*}$ denotes a hydrogen atom, a chlorine atom, a methyl group, a methoxy group, a nitro group or a trifluoromethyl group,

$R^{2*}$ denotes a hydrogen atom or a chlorine atom,

$R^{3*}$ denotes a hydrogen atom or a C1–C4-lower alkyl group,

$Y^*$ denotes the grouping $-O-(CH_2)_{m^*}-$,

$m^*$ denotes 0 or 1 and

$n^*$ denotes an integer from 3 to 7, the sum of $m^*$ and $n^*$ being an integer from 3 to 7,

and the salts of the carboxylic acids.

3. Phenoxy-substituted oxiranecarboxylic acids of the general formula I**

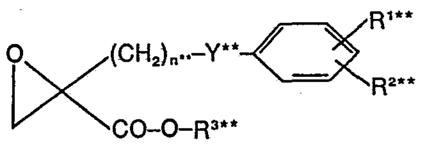

(I**)

wherein

$R^{1**}$ is in the meta- or para-position and

$R^{1**}$ denotes a hydrogen atom, a chlorine atom or a trifluoromethyl group,

$R^{2**}$ denotes a hydrogen atom,

$R^{3**}$ denotes a hydrogen atom, a methyl goup or an ethyl group,

$Y^{**}$ denotes the grouping –O–, and

$n^{**}$ denotes an integer from 4 to 6,

and the pharmacologically acceptable salts of the carboxylic acids.

4. Compound according to claim 3, selected from the group consisting of 2-[4-(3-chlorophenoxy)-butyl]-oxirane-2-carboxylic acid ethyl ester, 2-[4-(3-trifluoromethylphenoxy)-butyl]-oxirane-2-carboxylic acid ethyl ester, 2-[6-(4-chlorophenoxy)-hexyl]-oxirane-2-carboxylic acid ethyl ester and 2-[5-(4-chlorophenoxy)-pentyl]-oxirane-2-carboxylic acid ethyl ester, their corresponding oxirane-2-carboxylic acids and their pharmacologically acceptable salts.

5. Compound according to claim 3, wherein in formula I** $R^{1**}$ is a chlorine atom in para position, $R^{2**}$ denotes a hydrogen atom, $R^{3**}$ denotes an ethyl group, $Y^{**}$ denotes an oxygen atom and $n^{**}$ denotes 6.

6. Medicaments containing one or more compounds according to one of the claims 1 to 5.

7. Phen(alk)oxy-substituted oxiranecarboxylic acids of the general formula I, wherein the substituents have the meaning given in claim 1, for use in the treatment and prophylaxis of diseases based on disturbances of the glucuose and fat metabolism.

8. Process for the preparation of phen(alk)oxy-substituted oxiranecarboxylic acids of the general formula I

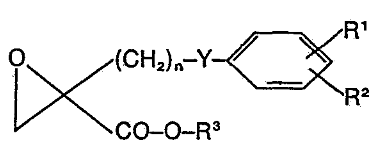

(I)

wherein

$R^1$ denotes a hydrogen atom, a halogen atom, a C1–C4-lower alkyl group, a C1–C4-lower alkoxy group, a nitro group or a trifluoromethyl group,

$R^2$ has one of the meanings of $R^1$,

$R^3$ denotes a hydrogen atom or a C1–C4-lower alkyl group,

Y denotes the grouping $-O-(CH_2)_m-$,

m denotes 0 or an integer from 1 to 4 and

n denotes an integer from 2 to 8, the sum of m and n being an integer from 2 to 8,

and of the salts of the acids, characterized in that substituted α-methylenecarboxylic acids of the general formula II

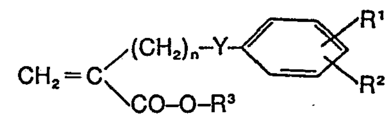

(II)

wherein

$R^1$, $R^2$, $R^3$, Y and n have the abovementioned meaning,

are oxidized and, thereafter, if desired, the resulting lower alkyl esters are saponified or the resulting acids are converted into salts or lower alkyl esters.

9. Process for the preparation of phen(alk)oxy-substituted oxiranecarboxylic acids of the general formula I*

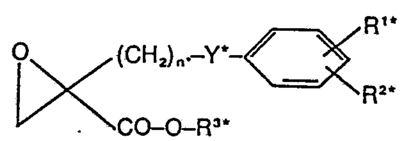

(I*)

wherein

$R^{1*}$ and $R^{2*}$ are in the meta- or para-position and

$R^{1*}$ denotes a hydrogen atom, a chlorine atom, a methyl group, a methoxy group, a nitro group or a trifluoromethyl group,

$R^{2*}$ denotes a hydrogen atom or a chlorine atom,

$R^{3*}$ denotes a hydrogen atom or a C1–C4-lower alkyl group,

$Y^*$ denotes the grouping $-O-(CH_2)_{m^*}-$,

$m^*$ denotes 0 or 1 and

$n^*$ denotes an integer from 3 to 7, the sum of $m^*$ and $n^*$ being an integer from 3 to 7,

and the salts of the acids, characterized in that substituted α-methylenecarboxylic acids of the general formula II*

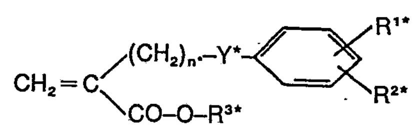

(II*)

wherein

$R^{1*}$, $R^{2*}$, $R^{3*}$, $Y^*$ and $n^*$ have the abovementioned meaning,

are oxidized and, thereafter, if desired, the resulting lower alkyl esters are saponified or the resulting acids are converted into salts or lower alkyl esters.

10. Process for the preparation of phenoxy-substituted oxiranecarboxylic acids of the general formula I**

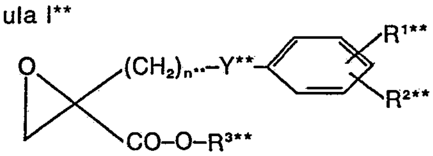

(I**)

wherein

$R^{1**}$ is in the meta- or para-position and

$R^{1**}$ denotes a hydrogen atom, a chlorine atom or a trifluoromethyl group,

$R^{2**}$ denotes a hydrogen atom,

$R^{3**}$ denotes a hydrogen atom, a methyl group or an ethyl group,

$Y^{**}$ denotes the grouping –O– and

$n^{**}$ denotes an integer from 4 to 6,

and the salts of the acids, characterized in that substituted α-methylenecarboxylic acids of the general formula II**

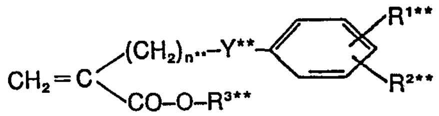

(II**)

wherein

$R^{1**}$, $R^{2**}$, $R^{3**}$, $Y^{**}$ and $n^{**}$ have the abovementioned meaning,

are oxidized and, thereafter, if desired, the resulting lower alkyl esters are saponified or the resulting acids are converted into salts or lower alkyl esters.

**Claims for the contracting state: AT**

1. Process for the preparation of phen(alk)oxy-substituted oxiranecarboxylic acids of the general formula I

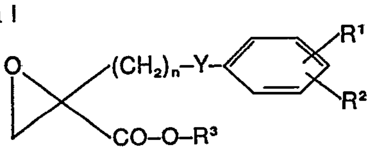

(I)

wherein

$R^1$ denotes a hydrogen atom, a halogen atom, a C1–C4-lower alkyl group, a C1–C4-lower alkoxy group, a nitro group or a trifluoromethyl group,

$R^2$ has one of the meanings of $R^1$,

$R^3$ denotes a hydrogen atom or a C1–C4-lower alkyl group,

Y denotes the grouping $-O-(CH_2)_m-$,

m denotes 0 or an integer from 1 to 4 and

n denotes an integer from 2 to 8, the sum of m and n being an integer from 2 to 8,

and of the salts of the acids, characterized in that substituted α-methylenecarboxylic acids of the general formula II

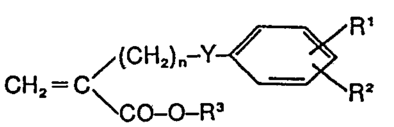

(II)

wherein

$R^1$, $R^2$, $R^3$, Y and n have the abovementioned meaning,

are oxidized and, thereafter, if desired, the resulting lower alkyl esters are saponified or the resulting acids are converted into salts or lower alkyl esters.

2. Process for the preparation of phen(alk)oxy-substituted oxiranecarboxylic acids of the general formula I*

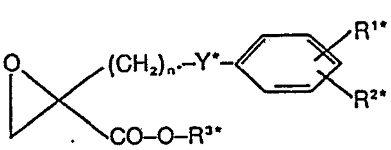

(I*)

wherein

$R^{1*}$ and $R^{2*}$ are in the meta- or para-position and

$R^{1*}$ denotes a hydrogen atom, a chlorine atom, a methyl group, a methoxy group, a nitro group or a trifluoromethyl group,

$R^{2*}$ denotes a hydrogen atom or a chlorine atom,

$R^{3*}$ denotes a hydrogen atom or a C1–C4-lower alkyl group,

$Y^*$ denotes the grouping $-O-(CH_2)_{m^*}-$,

$m^*$ denotes 0 or 1 and

$n^*$ denotes an integer from 3 to 7, the sum of $m^*$ and $n^*$ being an integer from 3 to 7,

and the salts of the acids, characterized in that substituted α-methylenecarboxylic acids of the general formula II*

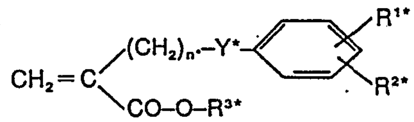

(II*)

wherein

$R^{1*}$, $R^{2*}$, $R^{3*}$, $Y^*$ and $n^*$ have the abovementioned meaning,

are oxidized and, thereafter, if desired, the resulting lower alkyl esters are saponified or the resulting acids are converted into salts or lower alkyl esters.

3. Process for the preparation of phenoxy-substituted oxiranecarboxylic acids of the general formula I**

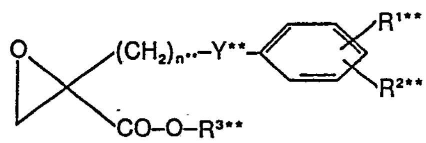

(I**)

wherein

$R^{1**}$ is in the meta- or para-position and

$R^{1**}$ denotes a hydrogen atom, a chlorine atom or a trifluoromethyl group,

$R^{2**}$ denotes a hydrogen atom,

$R^{3**}$ denotes a hydrogen atom, a methyl group or an ethyl group,

$Y^{**}$ denotes the grouping –O– and

$n^{**}$ denotes an integer from 4 to 6,

and the salts of the acids, characterized in that substituted α-methylenecarboxylic acids of the general formula II**

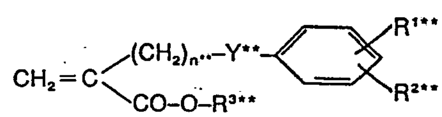

(II**)

wherein

$R^{1**}$, $R^{2**}$, $R^{3**}$, $Y^{**}$ and $n^{**}$ have the abovementioned meaning,

are oxidized and, thereafter, if desired, the resulting lower alkyl esters are saponified or the resulting acids are converted into salts or lower alkyl esters.

4. Process according to claim 3, characterized in that a compound of formula I**, selected from the group consisting of 2-[4-(3-chlorophenoxy)-butyl]-oxirane-2-carboxylic acid ethyl ester,

2-[4-(3-trifluoromethyl-phenoxy)-butyl]-oxirane-2-carboxylic acid ethyl ester, 2-[6-(4-chlorophenoxy)-hexyl]-oxirane-2-carboxylic acid ethyl ester and 2-[5-(4-chlorophenoxy)-pentyl]-oxirane-2-carbboxylic acid ethyl ester, their corresponding oxirane-2-carboxylic acids and their pharmacologically acceptable salts is prepared.

5. Process according to claim 3, characterized in that the compound of formula $I^{**}$, wherein $R^{1**}$ is a chlorine atom in para position, $R^{2**}$ denotes a hydrogen atom, $R^{3**}$ denotes an ethyl group, $Y^{**}$ denotes an oxygen atom and $n^{**}$ denotes 6, is prepared.

6. Process for the preparation of compositions with hypoglycemic and hypoketonemic action, characterized in that phen(alk)oxy-substituted oxiranecarboxylic acids of the general formula I

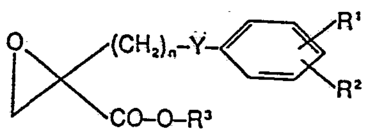
(I)

wherein

$R^1$ denotes a hydrogen atom, a halogen atom, a C1–C4-lower alkyl group, a C1–C4-lower alkoxy group, a nitro group or a trifluoromethyl group,

$R^2$ has one of the meanings of $R^1$,

$R^3$ denotes a hydrogen atom or a C1–C4-lower alkyl group,

Y denotes the grouping $-O-(CH_2)_m-$,

m denotes 0 or an integer from 1 to 4 and

n denotes an integer from 2 to 8, the sum of m and n being an integer from 2 to 8,

or their pharmacologically acceptable salts are prepared in that substituted α-methylenecarboxylic acids of the general formula II

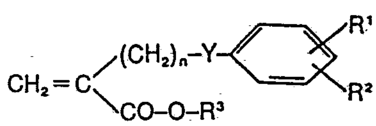
(II)

wherein

$R^1$, $R^2$, $R^3$, Y and n have the abovementioned meaning,

are oxidized and, thereafter, if desired, the resulting lower alkyl esters are saponified or the resulting acids are converted into salts or lower alkyl esters and that the compounds so obtained are brought into a form suitable for administration as hypoglycemic and hypoketonemic active composition by mixing them with suitable pharmaceutically compatible carriers.

**Revendications pour les Etats contractants: BE, CH, Li, DE, FR, GB, IT, LU, NL, SE**

1. Acides oxirannecarboxyliques substitués par un groupe phén(alk)oxy de formule générale I

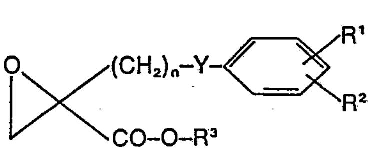
(I),

dans laquelle

$R^1$ est un atome d'hydrogène, un atome d'halogène, un groupe alkyle inférieur en C1–C4, un groupe alkoxy inférieur en C1–C4, un groupe nitro ou un groupe trifluorométhyle,

$R^2$ a l'une des significations de $R^1$,

$R^3$ est un atome d'hydrogène ou un groupe alkyle inférieur en C1–C4,

Y est le groupement $-O-(CH_2)_m-$,

m est 0 ou un nombre entier de 1 à 4 et

n est un nombre entier de 2 à 8,

la somme de m et n étant un nombre entier de 2 à 8, et les sels des acides carboxyliques.

2. Acides oxirannecarboxyliques substitués par un groupe phén(alk)oxy de formule générale $I^*$

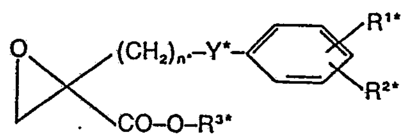
($I^*$),

dans laquelle $R^{1*}$ et $R^{2*}$ sont en position méta ou para et

$R^{1*}$ est un atome d'hydrogène, un atome de chlore, un groupe méthyle, un groupe méthoxy, un groupe nitro ou un groupe trifluorométhyle,

$R^{2*}$ est un atome d'hydrogène ou un atome de chlore,

$R^{3*}$ est un atome d'hydrogène ou un groupe alkyle inférieur en C1–C4,

$Y^*$ est le groupement $-O-(CH_2)_{m^*}-$,

$m^*$ est égal à 0 ou 1 et

$n^*$ est un nombre entier de 3 à 7,

la somme de $m^*$ et $n^*$ étant un nombre entier de 3 à 7, et les sels des acides carboxyliques.

3. Acides oxirannecarboxyliques substitués par un groupe phénoxy de formule générale $I^{**}$

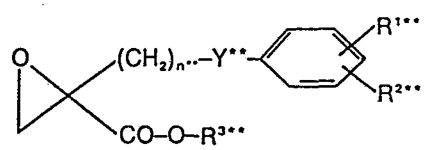
($I^{**}$),

dans laquelle $R^{1**}$ est en position méta ou para,

$R^{1**}$ est un atome d'hydrogène, un atome de chlore ou un groupe trifluorométhyle,

$R^{2**}$ est un atome d'hydrogène,

$R^{3**}$ est un atome d'hydrogène, un groupe méthyle ou un groupe éthyle,

$Y^{**}$ est le groupement –O–,

$n^{**}$ est un nombre entier de 4 à 6,

et les sels pharmaceutiquement acceptables des acides carboxyliques.

4. Composé suivant la revendication 3, choisi dans le groupe comprenant les composés suivants: 2-[4-(3-chlorophénoxy)-butyl]-oxiranne-2-carboxylate d'éthyle, 2-[4-(3-trifluorométhylphénoxy)-butyl]-oxiranne-2-carboxylate d'éthyle, 2-[6-(4-chlorophénoxy)-hexyl]-oxiranne-2-carboxylate d'éthyle, 2-[5-(4-chlorophénoxy)-pentyl]-oxiranne-2-carboxylate d'éthyle, les acides oxiranne-2-carboxyliques correspondants, ainsi que leurs sels pharmaceutiquement acceptables.

5. Composé suivant la revendication 3 dans lequel, dans la formule $1^{**}$, $R^{1**}$ est un atome de

chlore en position para, $R^{2**}$ est un atome d'hydrogène, $R^{3**}$ est un groupe éthyle, $Y^{**}$ est un atome d'oxygène et $n^{**}$ est égal à 6.

6. Médicament contenant un ou plusieurs composés suivant l'une quelconque des revendications 1 à 5.

7. Acides oxirannecarboxyliques substitués par un groupe phén(alk)oxy de formule générale I, dans lesquels les substituants ont la signification indiquée dans la revendication 1, destinés à être utilisés pour le traitement et la prophylaxie de maladies reposant sur des dérangements du métabolisme du glucose et des graisses.

8. Procédé pour la préparation d'acides oxiranne-carboxyliques substitués par un groupe phén(alk)oxy de formule générale I

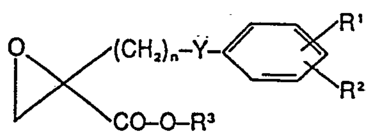 (I),

dans laquelle

$R^1$ est un atome d'hydrogène, un atome d'halogène, un groupe alkyle inférieur en C1–C4, un groupe alkoxy inférieur en C1–C4, un groupe nitro ou un groupe trifluorométhyle,

$R^2$ a l'une des significations de $R^1$,

$R^3$ est un atome d'hydrogène ou un groupe alkyle inférieur en C1–C4,

Y est le groupement $-O-(CH_2)_m-$,

m est égal à 0 ou est un nombre entier de 1 à 4 et

n est un nombre entier de 2 à 8,

la somme de m et n étant un nombre entier de 2 à 8, et des sels des acides, caractérisé en ce qu'on oxyde un acide α-méthylènecarboxylique substitué de formule générale II

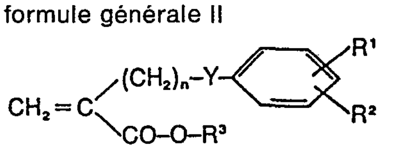 (II),

dans laquelle $R^1$, $R^2$, $R^3$, Y et n ont la signification indiquée ci-dessus, puis éventuellement on saponifie les esters d'alkyle inférieur obtenus ou bien on convertit les acides obtenus en sels ou en esters d'alkyle inférieur.

9. Procédé pour la préparation d'acides oxiranne-carboxyliques substitués par un groupe phén(alk)oxy de formule générale I*

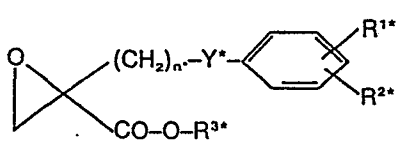 (I*),

dans laquelle $R^{1*}$ et $R^{2*}$ sont en position méta ou para,

$R^{1*}$ est un atome d'hydrogène, un atome de chlore, un groupe méthyle, un groupe méthoxy, un groupe nitro ou un groupe trifluorométhyle,

$R^{2*}$ est un atome d'hydrogène ou un atome de chlore,

$R^{3*}$ est un atome d'hydrogène ou un groupe alkyle inférieur en C1–C4,

$Y^*$ est le groupement $-O-(CH_2)_{m^*}-$,

$m^*$ est égal à 0 ou 1,

$n^*$ est un nombre entier de 3 à 7,

la somme de $m^*$ et $n^*$ étant un nombre entier de 3 à 7, et des sels des acides, caractérisé en ce qu'on oxyde un acide α-méthylènecarboxylique substitué de formule générale II*

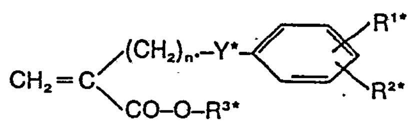 (II*),

dans laquelle $R^{1*}$, $R^{2*}$, $R^{3*}$, $Y^*$ et $n^*$ ont la signification indiquée ci-dessus, puis éventuellement on saponifie les esters d'alkyle inférieur obtenus ou on convertit les acides obtenus en sels ou en esters d'alkyle inférieur.

10. Procédé pour la préparation d'acides oxiranne-carboxyliques substitués par un groupe phénoxy de formule générale I**

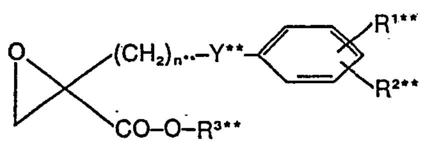 (I**),

dans laquelle $R^{1**}$ est en position méta ou para et

$R^{1**}$ est un atome d'hydrogène, un atome de chlore ou un groupe trifluorométhyle,

$R^{2**}$ est un atome d'hydrogène,

$R^{3**}$ est un atome d'hydrogène, un groupe méthyle ou un groupe éthyle,

$Y^{**}$ est le groupement $-O-$,

$n^{**}$ est un nombre entier de 4 à 6,

et des sels des acides, caractérisé en ce qu'on oxyde un acide α-méthylènecarboxylique substitué de formule générale II**

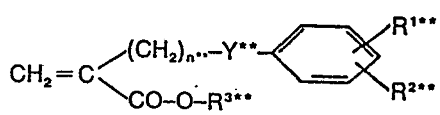 (II**),

dans laquelle $R^{1**}$, $R^{2**}$, $R^{3**}$, $Y^{**}$ et $n^{**}$ ont les significations indiquées ci-dessus, puis éventuellement on saponifie les esters d'alkyle inférieur obtenus ou on convertit les acides obtenus en sels ou en esters d'alkyle inférieur.

**Revendication pour l'Etat contractant: AT**

1. Procédé pour la préparation d'acides oxiranne-carboxyliques substitués par un groupe phén(alk)oxy de formule générale I

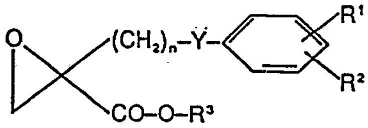 (I),

dans laquelle

$R^1$ est un atome d'hydrogène, un atome d'halogène, un groupe alkyle inférieur en C1–C4, un

groupe alkoxy inférieur en C1–C4, un groupe nitro ou un groupe trifluorométhyle,

$R^2$ a l'une des significations de $R^1$,

$R^3$ est un atome d'hydrogène ou un groupe alkyle inférieur en C1–C4,

Y est le groupement $-O-(CH_2)_m-$,

m est égal à 0 ou est un nombre entier de 1 à 4 et

n est un nombre entier de 2 à 8,

la somme de m et n étant un nombre entier de 2 à 8, et des sels des acides, caractérisé en ce qu'on oxyde un acide α-méthylènecarboxylique substitué de formule générale II

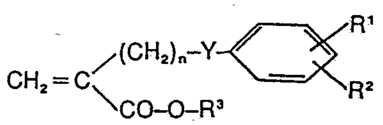 (II),

dans laquelle $R^1$, $R^2$, $R^3$, Y et n ont la signification indiquée ci-dessus, puis éventuellement on saponifie les esters d'alkyle inférieur obtenus ou bien on convertit les acides obtenus en sels ou en esters d'alkyle inférieur.

2. Procédé pour la préparation d'acides oxiranne-carboxyliques substitués par un groupe phén(alk)oxy de formule générale I*

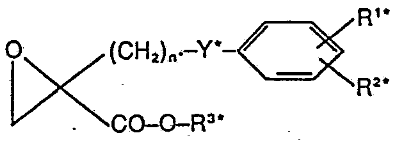 (I*),

dans laquelle $R^{1*}$ et $R^{2*}$ sont en position méta ou para,

$R^{1*}$ est un atome d'hydrogène, un atome de chlore, un groupe méthyle, un groupe méthoxy, un groupe nitro ou un groupe trifluorométhyle,

$R^{2*}$ est un atome d'hydrogène ou un atome de chlore,

$R^{3*}$ est un atome d'hydrogène ou un groupe alkyle inférieur en C1–C4,

Y* est le groupement $-O-(CH_2)_{m^*}-$,

m* est égal à 0 ou 1,

n* est un nombre entier de 3 à 7,

la somme de m* et n* étant un nombre entier de 3 à 7, et des sels des acides, caractérisé en ce qu'on oxyde un acide α-méthylènecarboxylique substitué de formule générale II*

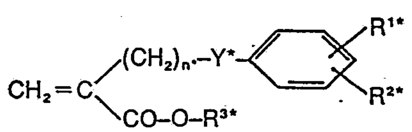 (II*),

dans laquelle $R^{1*}$, $R^{2*}$, $R^{3*}$, Y* et n* ont la signification indiquée ci-dessus, puis éventuellement on saponifie les esters d'alkyle inférieur obtenus ou on convertit les acides obtenus en sels ou en esters d'alkyle inférieur.

3. Procédé pour la préparation d'acides oxiranne-carboxyliques substitués par un groupe phénoxy de formule générale I**

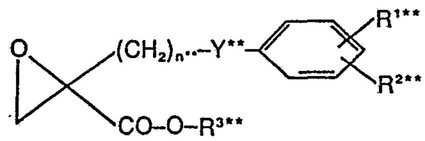 (I**),

dans laquelle $R^{1**}$ est en position méta ou para et

$R^{1**}$ est un atome d'hydrogène, un atome de chlore ou un groupe trifluorométhyle,

$R^{2**}$ est un atome d'hydrogène,

$R^{3**}$ est un atome d'hydrogène, un groupe méthyle ou un groupe éthyle,

Y** est le groupement –O–,

n** est un nombre entier de 4 à 6,

et des sels des acides, caractérisé en ce qu'on oxyde un acide α-méthylènecarboxylique substitué de formule générale II**

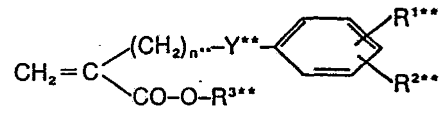 (II**),

dans laquelle $R^{1**}$, $R^{2**}$, $R^{3**}$, Y** et n** ont les significations indiquées ci-dessus, puis éventuellement on saponifie les esters d'alkyle inférieur obtenus ou on convertit les acides obtenus en sels ou en esters d'alkyle inférieur.

4. Procédé suivant la revendication 3, caractérisé en ce qu'on prépare un composé de formule I** choisi dans le groupe comprenant les composés suivants: 2-[4-(3-chlorophénoxy)-butyl]-oxiranne-2-carboxylate d'éthyle, 2-[4-(3-trifluorométhylphénoxy)-butyl]-oxiranne-2-carboxylate d'éthyle, 2-[6-(4-chlorophénoxy)-hexyl]-oxiranne-2-carboxylate d'éthyle, 2-[5-(4-chlorophénoxy)-pentyl]-oxiranne-2-carboxylate d'éthyle, les acides oxiranne-carboxyliques correspondants, ainsi que leurs sels pharmaceutiquement acceptables.

5. Procédé suivant la revendication 3, caractérisé en ce qu'on prépare le composé de formule I** dans lequel $R^{1**}$ est un atome de chlore en position para, $R^{2**}$ est un atome d'hydrogène, $R^{3**}$ est un groupe éthyle, Y** est un atome d'oxygène et n** est égal à 6.

6. Procédé pour la préparation d'agents ayant une action hypoglycémique et hypocétonémique, caractérisé en ce qu'on prépare des acides oxiranne-carboxyliques substitués par un groupe phényl(alk)oxy de formule générale I

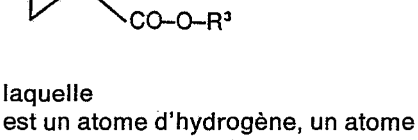 (I),

dans laquelle

$R^1$ est un atome d'hydrogène, un atome d'halogène, un groupe alkyle inférieur en C1–C4, un groupe alkoxy inférieur en C1–C4, un groupe nitro ou un groupe trifluorométhyle,

$R^2$ a l'une des significiations de $R^1$,

$R^3$ est un atome d'hydrogène ou un groupe alkyle inférieur en C1–C4,

Y est le groupement $-O-(CH_2)_m-$,

m est égal à 0 ou est un nombre entier de 1 à 4, et

n est un nombre entier de 2 à 8,

la somme de m et n étant un nombre entier de 2 à 8, ou leurs sels pharmaceutiquement acceptables en oxydant un acide α-méthylènecarboxylique substitué de formule générale II

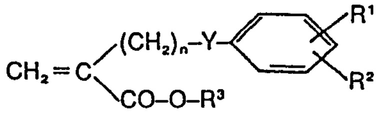 (II),

dans laquelle $R^1$, $R^2$, $R^3$, Y et n ont les significations indiquées ci-dessus, puis éventuellement on saponifie les esters d'alkyle inférieur obtenus ou on convertit les acides obtenus en sels ou en esters d'alkyle inférieur, et en ce qu'on amène l'un des composés ainsi obtenus sous une forme appropriée en vue d'une administration à titre d'agent ayant une action hypoglycémique et hypocétonémique par mélange avec un véhicule ou excipient pharmaceutiquement acceptable approprié.